# EUROPÄISCHE PATENTSCHRIFT

(11) **EP 3 852 601 B1**
(45) Veröffentlichungstag und Bekanntmachung des Hinweises auf die Patenterteilung: **02.11.2022**
(21) Anmeldenummer: 20739298.6
(22) Anmeldetag: 02.07.2020
(51) Int. Cl.: A61B 1/00, A61B 17/16, A61B 17/22, A61B 17/29, A61B 17/32

(54) **BEHANDLUNGSINSTRUMENT FÜR ENDOSKOP**
TREATMENT INSTRUMENT FOR ENDOSCOPE
INSTRUMENT DE TRAITEMENT POUR ENDOSCOPE

(30) Priorität: 04.07.2019 DE 102019118043
(43) Veröffentlichungstag der Anmeldung: 28.07.2021
(73) Patentinhaber: Ovesco Endoscopy AG, 72074 Tübingen (DE)
(72) Erfinder: HOFMANN, Nico, 73249 Wernau (DE); CONRAD, Gabor, 72074 Tübingen (DE); MEINING, Alexander Prof. Dr. med., 97070 Würzburg (DE); HO, Chi-Nghia, 72762 Reutlingen (DE)
(74) Vertreter: Winter, Brandl - Partnerschaft mbB
(86) Internationale Anmeldenummer: PCT/EP2020/068720
(87) Internationale Veröffentlichungsnummer: WO 2021/001499

(56) Entgegenhaltungen:
- EP-B1- 1 543 786
- US-A- 5 172 700
- US-A1- 2016 073 859
- US-A1- 2019 125 165

## Beschreibung

### Technisches Gebiet

Die vorliegende Erfindung bezieht sich auf ein Behandlungsinstrument/Smartfunktionsträger/Nachrüstsatz, welches/welcher mit einem distalen Ende/Endabschnitt/Endoskopkopf eines Endoskops koppelbar (z.B. außenumfangsseitig aufsteckbar) ist und welches/welcher einen Hohlkörper (Koppelabschnitt) sowie ein Greiforgan (Effektorabschnitt) aufweist, wobei das Greiforgan (Effektorabschnitt) über eine innerhalb des Hohlkörpers (Koppelabschnitt) befindliche Stellmechanik betätigbar ist. Des Weiteren bezieht sich die vorliegende Erfindung auf ein Endoskop mit distaler Optik und ggf. Arbeitskanal sowie mit einem distalen Ende/Endabschnitt/Endoskopkopf, welches/welcher mit dem Behandlungsinstrument gekoppelt ist.

### Stand der Technik

Ein gattungsgemäßes Behandlungsinstrument/Smartfunktionsträger/Nachrüstsatz sind beispielsweise aus der EP 1 543 786 B1, US5172700 und US2016/073859 bekannt. Diese haben einen kappen- oder hülsenförmigen proximalen (dem behandelnden Arzt zugewandten) Aufsteckabschnitt und einen distalen (vom behandelnden Arzt abgewandten) Effektorabschnitt, der mittels Seilzügen betätigbar ist. Bei Untersuchungen menschlicher Körper mittels eines mit dem gattungsgemäßen Behandlungsinstrument ausgestatteten/nachgerüsteten Endoskops hat sich jedoch gezeigt, dass eine distale Optik des Endoskops am Endoskopkopf besonders anfällig für Verschmutzungen ist, welche die Sicht des behandelnden Arztes (eines Operateurs bzw. Bedieners) beeinträchtigen können.

Die Anmelderin hat festgestellt, dass diese Verschmutzungstendenz daraus resultiert, dass Verschmutzungen auf der Optik durch eine bei Endoskopen übliche Reinigungsvorrichtung/Spüldüse nicht mehr ausreichend entfernt /abgespült werden kann, da sie sich innerhalb des hülsenförmigen Kopplungsabschnitts sammelt.

Vor diesem Hintergrund ist es eine Aufgabe der vorliegenden Offenbarung, ein Behandlungsgerät/Smartfunktionsträger/Nachrüstsatz der vorstehend genannten Gattung für ein bzw. zur adaptiven/wahlweisen Montage an/auf einem Endoskop bereitzustellen, welches die Wahrscheinlichkeit für Bedienersicht-einschränkende Verschmutzungen der (distalen) Endoskop-Optik verringert.

Diese Aufgabe wird durch ein Behandlungsinstrument bzw. Behandlungsgerät/Smartfunktionsträger/Nachrüstsatz mit den Merkmalen des Anspruchs 1 und durch ein Endoskop (mit Optik und ggf. Arbeitskanal) mit den Merkmalen des Anspruchs 14 oder 15 gelöst. Vorteilhafte Weiterbildungen des Behandlungsinstruments sind Gegenstand der Unteransprüche.

Ein offenbarungsgemäßes Behandlungsgerät/Smartfunktionsträger/Nachrüstsatz ist mit einem distalen Ende/Endabschnitt/Endoskopkopf eines Endoskops (mit Optik und ggf. internem Arbeitskanal im Endoskopschaft) koppelbar und weist einen Hohlkörper/Kopplungsabschnitt (Hülse) sowie ein Greiforgan/Effektorabschnitt/Effektor (vorzugsweise aus zwei Zangen- oder Pinzettenbranchen) auf. Das Greiforgan ist über eine Stellmechanik (Betätigungszüge) betätigbar. Ein distales Ende bzw. ein distaler Endabschnitt der Stellmechanik befindet sich innerhalb des Hohlkörpers. Insbesondere befindet sich das distale Ende/Endabschnitt der Stellmechanik innerhalb einer Aussparung in einer (umfangsseitigen) Außenwand des Hohlkörpers. Von dem Hohlkörper aus erstreckt sich die Stellmechanik bzw. ein proximaler Abschnitt der Stellmechanik in eine proximale (dem behandelnden Arzt zugewandte) Richtung.

Anders ausgedrückt weist das erfindungsgemäße Behandlungsinstrument mit dem Hohlkörper ein Befestigungsteil auf, welches an einem proximalen Ende/Endbereich des Behandlungsinstruments einen Befestigungs-/Aufsteckabschnitt aufweist, der geeignet und dafür vorgesehen ist, ein distales Ende/Endabschnitt/Endoskopkopfs eines Einführabschnitts (Schaft) eines Endoskops aufzunehmen, um das Behandlungsinstrument an dem distalen Ende/Endabschnitt des Einführabschnitts/Schafts des Endoskops zu befestigen. Das Greiforgan dient als ein Behandlungsteil, um ein gegebenenfalls distal vom Befestigungsabschnitt angeordnetes Gewebe bzw. distal vom Befestigungsabschnitt befindliches Objekt, beispielsweise ein Fremdkörper oder Bolus, mittels Verstellen des Behandlungsteils bzw. des Greiforgans behandeln bzw. ergreifen zu können. Um dieses Verstellen des Greiforgans zu bewirken, ist die Stellmechanik, insbesondere ein distales Ende/Endabschnitt der Stellmechanik, unmittelbar oder mittelbar mit dem Greiforgan, insbesondere mit einem proximalen Ende/Endabschnitt des Greiforgans, verbunden.

Konstruktiver ausgedrückt hat das Behandlungsinstrument gemäß der vorliegenden Offenbarung
- eine Hülse (Grundkörper), die an ihrem proximalen Endabschnitt (Koppelabschnitt) dafür vorgesehen und ausgebildet ist, an/auf einen Endoskopschaft oder Endoskopkopf angesetzt/aufgesteckt zu werden,
- ein Greiforgan, beispielsweise zwei Zangen- oder Pinzettenbranchen, das an einem distalen Endabschnitt (Effektorabschnitt) der Hülse (schwenkbar) gelagert ist und
- Betätigungszüge, die an dem Greiforgan (Zangen- oder Pinzettenbranchen) befestigt sind (sowie vorzugsweise durch die Hülse verlaufen) und über die zumindest Schließ-/Greifkräfte (und vorzugsweise auch Öffnungskräfte) auf das Greiforgan (Zangen- oder Pinzettenbranchen) zu dessen entsprechender Betätigung übertragbar/aufbringbar sind.

Das offenbarungsgemäße Behandlungsinstrument zeichnet sich insbesondere dadurch aus, dass das Greiforgan (in geschlossener Position/Greifposition) in Form einer Kappe oder Klappe ausgebildet ist bzw. eine Kappenform ausbildet (d.h. die Zangen- oder Pinzettenbranchen sind so, vorzugsweise schalen- oder klappenartig geformt und dimensioniert, dass sie in Greifposition eine zumindest in Richtung distal geschlossene, vorzugsweise halbkugelförmige Kappe oder Klappe bilden), die so dimensioniert ist, dass sie ein distales Ende des Hohlkörpers (offene distale Stirnsseite der Hülse) verschließt. Außerdem ist das Greiforgan derart ausgebildet, dass es in Freigabeposition eine Stirnfläche des Hohlkörpers vollständig oder nahezu vollständig in distaler Richtung freigibt.

Unter "verschlossen" wird in diesem Zusammenhang ein Zustand verstanden, in welchem eine Struktur, bzw. im vorliegenden Fall das Greiforgan, zumindest für Gewebepartikel, insbesondere für Gewebepartikel mit einem minimalen Außendurchmesser von 1,1 mm zumindest weitgehend undurchlässig ist. Mit anderen Worten ist das Greiforgan derart ausgebildet, dass es in einer verschlossenen Stellung (Greifposition) zumindest keine Aussparung mit einer maximalen Öffnungsweite von 1,1 mm oder mehr und/oder zumindest keinen Spalt mit einem maximalen Spaltmaß von 1,1 mm oder mehr aufweist.

Insbesondere wird unter "verschlossen" ein Zustand verstanden, in welchem das Greiforgan des mit einem distalen Ende/Endabschnitt eines Endoskops gekoppelten Behandlungsinstruments in einer verschlossenen Stellung (Greifposition) ein an dem distalen Ende des Endoskops angeordnetes Objektiv einer Optik des Endoskops in einer distalen Richtung verdeckt.

Anders ausgedrückt zeichnet sich das Behandlungsinstrument dadurch aus, dass es ausgebildet ist, eine distale Stirnfläche des Hohlkörpers bzw. des Befestigungsabschnitts in einer geschlossenen Stellung des Greiforgans in distaler Richtung zumindest weitgehend, vorzugsweise vollständig zu verschließen.

Unter der Bezeichnung "Greifposition" ist die konstruktive Position zu verstehen, welche die Mechanik des Greiforgans oder die Stellmechanik (konstruktiv) maximal zulässt. D.h., beispielsweise im Fall einer Fasszange oder Pinzette als Greiforgan definiert sich die Greifposition konstruktiv als jene maximale Position (Konstruktionslage), in welcher die Zangen- oder Pinzettenbranchen aneinander anliegen oder mittels eines ggf. vorgesehenen Anschlags (im Greiforgan oder in der Stellmechanik) mit einem Minimalspalt voneinander beabstandet sind. Gleiches gilt vorzugsweise auch für die Freigabeposition, welche jene konstruktive Position (Konstruktionslage) betrifft/betreffen kann, die durch die Mechanik des Greiforgans oder durch die Stellmechanik beispielsweise per Anschlag zugelassen wird, wobei an dieser Stelle darauf hingewiesen werden soll, dass insbesondere die Freigabeposition auch undefiniert (d.h. beliebig) sein kann.

Durch die Ausbildung des Greiforgans als Kappe/Klappe kann in vorteilhafter Weise die Wahrscheinlichkeit für Verschmutzungen einer Optik eines mit dem erfindungsgemäßen Behandlungsinstrument ausgestatteten Endoskops verringert werden.

Das Greiforgan kann zumindest abschnittsweise transparent ausgebildet bzw. lichtdurchlässig sein. Insbesondere kann das Greiforgan derart ausgebildet sein, dass es zumindest abschnittsweise transparent bzw. lichtdurchlässig in Bezug auf Licht des für einen Menschen sichtbaren Spektrums ist (Licht mit einer Wellenlänge zwischen 420 und 750 nm). Alternativ oder zusätzlich kann das Greiforgan zumindest abschnittsweise auch für infrarotes oder ultraviolettes Licht durchlässig ausgebildet sein.

Ein zumindest abschnittsweise transparent ausgebildetes Greiforgan ermöglicht in vorteilhafter Weise, dass eine Optik eines mit einem erfindungsgemäßen Behandlungsinstrument ausgestatteten Endoskops zumindest teilweise auch dann funktionstüchtig ist, wenn die Optik durch das Greiforgan verschlossen ist.

Das Greiforgan (d.h. die Zangen-/Pinzettenbranchen) kann an einer Stirnseite einen flachen Plattenabschnitt oder einen einfach gekrümmten Schalenabschnitt aufweisen (oder ist/sind als flacher Plattenabschnitt bzw. Schalenabschnitt ausgebildet), welcher in einer geschlossenen Stellung des Greiforgans an einer distalen Seite des Greiforgans angeordnet ist. Alternativ kann das Greiforgan an einer Stirnfläche einen sich in Richtung hin zum Hohlkörper bzw. in proximale Richtung konisch aufweitenden Schalenabschnitt aufweisen, welcher in einer geschlossenen Stellung des Greiforgans an einer distalen Seite des Greiforgans angeordnet ist. Insbesondere erstreckt sich der flache Plattenabschnitt bzw. der (dreidimensional gekrümmte) Schalenabschnitt senkrecht zu einer Längserstreckungsachse des Hohlkörpers. Insbesondere ist das Greiforgan derart ausgebildet, dass der flache Plattenabschnitt bzw. der einfach gekrümmte Schalenabschnitt bzw. der sich in proximale Richtung konisch aufweitende Schalenabschnitt zumindest abschnittsweise transparent ist.

Durch Vorsehen des flachen Plattenabschnitts oder des einfach gekrümmten Schalenabschnitts kann in vorteilhafter Weise die Wahrscheinlichkeit für Gewebeverletzungen verringert werden, beispielsweise, wenn ein mit dem Behandlungsinstrument ausgestattetes Endoskop bei einer Untersuchung auf eine Striktur trifft. Durch Vorsehen des sich in proximale Richtung konisch aufweitenden Schalenabschnitts kann eine Striktur beim Passieren des Behandlungsinstruments auf schonende Weise aufgeweitet werden. Dies kann durch Öffnen des Greifinstruments während des Passierens des Behandlungsinstruments unterstützt werden. Ist der flache Plattenabschnitt bzw. der einfach gekrümmte Schalenabschnitt zumindest abschnittsweise transparent ausgebildet, ermöglicht die einfache Geometrie eine einfachere Berücksichtigung etwaig auftretender optischer Verzerrungen. Ist der sich in proximale Richtung aufweitende Schalenabschnitt transparent ausgebildet, kann ein Ansteuern einer zu passierenden Striktur erleichtert werden.

Ein Abschnitt eines Randes eines proximalen Endes des Greiforgans kann derart über ein Gelenk mit einem Abschnitt eines Randes eines distalen Endes des Hohlkörpers um eine Schwenkachse schwenkbeweglich verbunden sein, dass sich die Schwenkachse tangential zu beiden Abschnitten erstreckt. Insbesondere ist das Greiforgan derart schalenförmig ausgebildet, dass das proximale Ende des Greiforgans in der geschlossenen Stellung des Greiforgans bündig in das distale Ende des Hohlkörpers übergeht. Insbesondere kann das den Hohlkörper mit dem Greiforgan verbindende Gelenk am Hohlkörper in Form eines in distale Richtung vorragenden Fortsatzes vorgesehen sein.

In anderen Worten ausgedrückt sind die sich gegenüberliegenden Zangen- oder Pinzettenbranchen des Greiforgans an der distalen Stirnkante des Hohlkörpers/Hülse oder nahe zu dieser anscharniert, derart, dass die Zangen- oder Pinzettenbranchen in Greifposition (Schließposition) weitgehend bündig (ohne größere Überstände) zur Mantelfläche des Hohlkörpers/Hülse anschließen. Dadurch ergibt sich in Greifposition der kugelschalenförmigen Zangen- oder Pinzettenbranchen quasi eine Art Kuppel oder Dom, der längsumfänglich an der distalen Stirnkante des Hohlkörpers/Hülse mit möglichst geringem axialen Spaltmaß quasi anliegt, ohne über die Außenmantelfläche über- oder zurückzutreten. Dabei sei darauf hingewiesen, dass unter dem Begriff "anliegen" auch ein bestimmter Axialabstand zu verstehen ist, der in Bezug auf mögliches umfängliches Eindringen von Schmutz oder Flüssigkeit keine große Rolle spielt.

Durch die tangentiale Anordnung des den Hohlkörper mit dem Greiforgan verbindenden Gelenks/Scharniers kann das Greiforgan in vorteilhafter Weise derart ausgebildet werden, dass es in einer geöffneten Stellung eine Stirnfläche des Hohlkörpers vollständig oder nahezu vollständig in distaler Richtung freigibt.

Die Stellmechanik kann wenigstens ein in dem Hohlkörper verschiebbar gelagertes Stellglied aufweisen, welches beispielsweise über wenigstens einen Pleuel bzw. wenigstens eine Schubstange oder wenigstens ein flexibles Element mit dem Greiforgan verbunden ist. Anders ausgedrückt kann die Stellmechanik ein bezüglich des Hohlkörpers translatorisch bzw. vorwiegend translatorisch bewegliches bzw. gelagertes Stellglied (Stößel) aufweisen, dessen translatorische Bewegung über einen oder mehrere Pleuel bzw. über eine oder mehrere Schubstangen bzw. über ein oder mehrere flexible Elemente eine rotatorische Bewegung des Greiforgans bzw. von einem Teil bzw. von Teilen des Greiforgans bewirkt. Der/die Pleuel bzw. die Schubstange(n) kann/können mit dem Greiforgan bzw. einem Teil des Greiforgans (nämlich eine der oder beide Zangen-/Pinzettenbranchen) über ein Dreh-/Scharniergelenk schwenkbeweglich verbunden sein. Der Pleuel bzw. die Schubstange kann mit dem Stellglied über ein Drehgelenk schwenkbeweglich verbunden sein. Das flexible Element kann mit dem Greiforgan und mit dem Stellglied jeweils fest verbunden sein.

Wird das Stellglied in Richtung hin zum distalen Ende des Hohlkörpers bewegt, öffnet sich das Greiforgan. Umgekehrt schließt sich das Greiforgan, wenn das Stellglied in Richtung proximales Ende des Hohlkörpers bewegt wird.

Das flexible Element ist insofern flexibel, als es bei einem Verschieben des Stellglieds derart reversibel verformt wird, dass es eine Kraftübertragung bzw. eine Bewegungsübertragung von dem Stellglied auf das Greiforgan ermöglicht.

Der Pleuel kann derart über ein Gelenk mit einem (in Bezug auf den mit dem das Greiforgan bzw. ein Greifteil mit dem Hohlkörper verbindenden Gelenk versehenen Abschnitt) weiteren Abschnitt des Randes des proximalen Endes des, insbesondere schalenförmigen, Greiforgans bzw. Greifteils um eine Schwenkachse schwenkbeweglich verbunden sein, dass sich die Schwenkachse senkrecht zu dem weiteren Abschnitt erstreckt. Insbesondere kann sich die Schwenkachse des den Hohlkörper mit Greiforgan bzw. Greifteil verbindenden Gelenks parallel zu der Schwenkachse des das Greiforgan bzw. Greifteil mit dem Pleuel verbindenden Gelenks erstrecken. Insbesondere können das den Hohlkörper mit dem Greiforgan bzw. Greifteil verbindende Gelenk und das das Greiforgan bzw. Greifteil mit dem Pleuel verbindenden Gelenk an zwei unterschiedlichen, insbesondere sich gegenüberliegenden, Seiten des Greiforgans bzw. Greifteils angeordnet sein.

Durch diese Anordnung des Pleuels in Bezug auf das Greiforgan bzw. Greifteil ist es in vorteilhafter Weise möglich, einen von dem Hohlkörper und dem geschlossenen Greiforgan eingegrenzten Innenraum zu vergrößern.

Das Greiforgan kann zwei Greifteile (d.h. die Zangen-/Pinzettenbranchen) aufweisen. Eines der beiden Greifteile kann fest mit dem Hohlkörper verbunden sein. Das andere der beiden Greifteile kann schwenkbeweglich oder schubbeweglich mit dem Hohlkörper verbunden sein. Anders ausgedrückt kann das bewegliche Greifteil über ein Drehgelenk oder über ein Schubgelenk mit dem Hohlkörper verbunden sein.

Der zumindest eine Pleuel bzw. die zumindest eine Schubstange oder das zumindest eine flexible Element kann das mit dem Hohlkörper beweglich verbundene Greifteil mit dem Stellglied verbinden. Insbesondere können ein weiteres Stellglied und ein weiterer Pleuel bzw. eine weitere Schubstange oder ein weiteres flexibles Element vorgesehen sein, wobei der weitere Pleuel bzw. die weitere Schubstange oder das weitere flexible Element das mit dem Hohlkörper beweglich verbundene Greifteil mit dem weiteren Stellglied verbindet.

Ein distales Ende des Stellglieds und ein distales Ende des weiteren Stellglieds können insbesondere an zwei sich gegenüberliegenden Seiten des Hohlkörpers angeordnet sein.

Alternativ kann das Greiforgan zwei Greifteile aufweisen, welche beide schwenkbeweglich oder schubbeweglich mit dem Hohlkörper verbunden sind. Anders ausgedrückt können die beweglichen Greifteile jeweils über ein Drehgelenk oder über ein Schubgelenk mit dem Hohlkörper verbunden sein.

Der Pleuel bzw. die Schubstange oder das flexible Element kann das Stellglied mit einem der beiden Greifteile verbinden. Ein weiterer Pleuel bzw. eine weitere Schubstange oder ein weiteres flexibles Element kann vorgesehen sein, um das Stellglied auch mit dem anderen der beiden Greifteile zu verbinden. Insbesondere kann das Behandlungsinstrument auch ein weiteres Stellglied aufweisen, welches mit den beiden beweglichen Greifteilen jeweils über einen weiteren Pleuel bzw. eine weitere Schubstange oder ein weiteres flexibles Element verbunden ist. Wie bei der Konfiguration, in welcher nur ein Greifteil beweglich mit dem Hohlkörper verbunden ist, können auch bei der Konfiguration, in welcher beide Greifteile beweglich mit dem Hohlkörper verbunden sind, ein distales Ende des Stellglieds und ein distales Ende des weiteren Stellglieds an zwei sich gegenüberliegenden Seiten des Hohlkörpers angeordnet sein.

Das Stellglied und/oder das weitere Stellglied bzw. das distale Ende des Stellglieds und/oder das distale Ende des weiteren Stellglieds können/kann sich symmetrisch zu/an/in einer Ebene erstrecken, welche sich mittig zwischen zwei Greifteilen des Greiforgans erstreckt. Anders ausgedrückt kann das Greiforgan in der Art eines Zweischalengreifers eines Baggers ausgebildet sein.

Insbesondere können die beiden beweglich mit dem Hohlkörper verbundenen Greifteile derart sich gegenüberliegend angeordnet sein, dass sich das Stellglied und/oder das weitere Stellglied bzw. das distale Ende des Stellglieds und/oder das distale Ende des weiteren Stellglieds mittig zwischen den beiden Greifteilen erstrecken/erstreckt.

Ist nur eines von zwei Greifteilen schwenkbeweglich mit dem Hohlkörper verbunden, kann die Herstellung des Behandlungsinstruments verhältnismäßig einfach gehalten werden. Zwei schwenkbeweglich mit dem Hohlkörper verbundene Greifteile vorzusehen, kann insofern vorteilhaft sein, als das Behandlungsinstrument derart ausgebildet werden kann, dass Gewebe, welches von dem Behandlungsinstrument ergriffen werden soll, vor dem Greifen besser einsehbar sein kann.

Gemäß einem Aspekt der Erfindung kann das Behandlungsinstrument bzw. das Greiforgan mehr als zwei Greifteile aufweisen. Alle Greifteile können schwenkbeweglich oder schubbeweglich mit dem Hohlkörper verbunden sein. Anders ausgedrückt können alle Greifteile jeweils über zumindest ein Drehgelenk oder zumindest ein Schubgelenk mit dem Hohlkörper verbunden sein. Alternativ ist es auch möglich, zumindest eines der Greifteile des Behandlungsinstruments mit mehr als zwei Greifteilen derart auszubilden, dass es fest mit dem Hohlkörper verbunden ist.

Das Behandlungsinstrument mit mehr als zwei Greifteilen kann derart ausgebildet sein, dass jedes der schwenk- oder schubbeweglich mit dem Hohlkörper verbundenen Greifteile jeweils über ein eigens vorgesehenes Stellglied ansteuerbar ist. Anders ausgedrückt, kann das Behandlungsinstrument so viele Stellglieder wie schwenk- oder schubbeweglich mit dem Hohlkörper verbundene Greifteile aufweisen. Dabei kann jedes Stellglied jeweils über einen (eigenen) Pleuel bzw. eine (eigene) Schubstange oder ein (eigenes) flexibles Element mit dem entsprechenden Greifteil verbunden sein. Alternativ ist es auch möglich, das Behandlungsinstrument derart auszubilden, dass mehrere der schwenk- oder schubbeweglich mit dem Hohlkörper verbundenen Greifteile mittels eines gemeinsamen Stellglieds ansteuerbar sind. Anders ausgedrückt, kann das gemeinsame Stellglied mittels mehrerer Pleuel bzw. Schubstangen oder mehrerer flexibler Elemente mit mehreren Greifteilen verbunden sein, so dass ein Verschieben des gemeinsamen Stellglieds ein Verstellen mehrerer Greifteile bewirkt. Es ist auch möglich, das mehr als zwei Greifteile aufweisende Behandlungsinstrument mit mehreren gemeinsamen Stellgliedern auszubilden, das heißt, mit mehreren Stellgliedern, mittels derer jeweils mehrere Greifteile ansteuerbar sind.

Die distalen Enden der Stellglieder können der Anzahl und der Anordnung der Greifteile entsprechend, in den Seiten des Hohlkörpers angeordnet sein. Insbesondere kann die Anordnung der distalen Enden der Stellglieder so ausgelegt sein, dass die Pleuel bzw. die Schubstangen oder die flexiblen Elemente die mit dem Hohlkörper beweglich verbundenen Greifteile jeweils zentral in Bezug auf das jeweilige Greifteil mit dem Stellglied verbinden. Diese Art der Anordnung ist insofern wünschenswert, dass dadurch keine bzw. nur geringe zusätzlichen Kräfte zu den Kräften, die die Bewegung des Greifteils bzw. der Greifteile ermöglichen, auftreten, die beispielsweise zu Windungen in dem/den Gelenke/n führen können.

Das Greiforgan bzw. zumindest eines der Greifteile kann eine Aussparung mit einer maximalen Öffnungsweite von 1,1 mm oder weniger aufweisen. Insbesondere kann die Aussparung derart an dem Greiforgan angeordnet sein, dass sie sich in der geschlossenen Stellung des Greiforgans an der distalen Seite bzw. an der Stirnseite des Greiforgans in einem mittigen bzw. zentralen Bereich befindet. Insbesondere kann die Aussparung derart ausgebildet sein, dass sie sich zu gleichen oder unterschiedlichen Teilen in den das Greiforgan bildenden Greifteilen erstreckt. Insbesondere erstreckt sich die Aussparung in der bevorzugten Ausführung mit zwei Greifteilen teils in dem einen der beiden Greifteile und teils in dem anderen der beiden Greifteile. Insbesondere kann die Aussparung in Form einer runden bzw. kreiszylindrischen Öffnung vorgesehen sein.

Durch Vorsehen einer Aussparung mit einer maximalen Öffnungsweite von 1,1 mm oder weniger ist es in vorteilhafter Weise möglich, durch das sich in der geschlossenen Stellung befindlichen Greiforgan einen Führungsdraht hindurch zu schieben. Durch die Limitierung des Ausmaßes der Aussparung kann weiterhin in vorteilhafter Weise ein Durchdringen des Greiforgans durch störendes Gewebe verhindert werden.

Ein erfindungsgemäßes Endoskop weist ein distales Ende auf, welches mit einem erfindungsgemäßen Behandlungsinstrument gekoppelt ist. Ein proximaler Abschnitt der Stellmechanik des Behandlungsinstruments ist entlang einer Außenseite des Endoskops geführt.

Ein alternativer oder zusätzlicher Aspekt der vorliegenden Erfindung betrifft ein Endoskop mit einem distalen Ende, welches an einer Stirnfläche ein Objektiv einer Optik aufweist und welches mit einem Behandlungsinstrument gekoppelt ist. Das Behandlungsinstrument weist einen Hohlkörper sowie ein Greiforgan auf. Das Greiforgan ist über eine Stellmechanik betätigbar, welche ein sich innerhalb des Hohlkörpers befindliches distales Ende aufweist. Die Stellmechanik erstreckt sich von dem Hohlkörper aus in eine proximale Richtung und weist einen proximalen Abschnitt auf, der entlang einer Außenseite des Endoskops geführt werden kann.

Das Greiforgan ist in Form einer Kappe ausgebildet, welche in eine geschlossene Stellung bringbar ist. Das Greiforgan ist derart ausgebildet, dass das Greiforgan bzw. ein Teil des Greiforgans in der geschlossenen Stellung des Greiforgans das Objektiv der Optik des Endoskops in distaler Richtung verdeckt, wenn das Behandlungsinstrument mit dem distalen Ende des Endoskops gekoppelt ist. Zudem gibt das Greiforgan in Freigabeposition eine Stirnfläche des Hohlkörpers vollständig oder nahezu vollständig in distaler Richtung frei.

Somit kann in vorteilhafter Weise die Funktionalität des Greiforgans erweitert werden, da es nicht nur zum Greifen, sondern auch zum Verdecken des Objektivs genutzt werden kann.

Das Endoskop kann einen Spülkanal aufweisen, durch welchen Spülflüssigkeit zu einer an der Stirnfläche des Endoskops angeordneten Spülkanalaustrittsöffnung gefördert werden kann. Das Greiforgan kann derart ausgebildet sein, dass das Greiforgan oder ein Teil des Greiforgans in der geschlossenen Stellung des Greiforgans die Spülkanalaustrittsöffnung zumindest teilweise in distaler Richtung verdeckt, wenn das Behandlungsinstrument mit dem distalen Ende des Endoskops gekoppelt ist. Das Greiforgan kann darüber hinaus derart ausgebildet sein, dass eine in proximale Richtung weisende Innenfläche des Greiforgans in der geschlossenen Stellung des Greiforgans derart von dem Objektiv der Optik und von der Spülkanalaustrittsfläche beabstandet ist, dass in einem Zustand, in welchem das Behandlungsinstrument mit dem distalen Ende des Endoskops gekoppelt ist, in welchem das Greiforgan geschlossen ist und in welchem Spülflüssigkeit zu der Spülkanalaustrittsöffnung gefördert wird, Spülflüssigkeit, das heißt zumindest ein Teil der zu der Spülkanalaustrittsöffnung gefördert Spülflüssigkeit, nach einem Austreten aus der Spülkanalaustrittsöffnung auf die in proximale Richtung weisende Innenfläche des Greiforgans auftrifft und auf das Objektiv der Optik reflektiert wird.

Somit kann Spülflüssigkeit nicht nur zum Spülen von distal vom Endoskop angeordnetem Gewebe, sondern auch zum Reinigen des Objektivs der Optik des Endoskops eingesetzt werden.

### Kurzbeschreibung der Zeichnungen

Die vorliegende Erfindung wird im Folgenden anhand bevorzugter Ausführungsbeispiele unter Bezugnahme auf die beigefügten Zeichnungen näher beschrieben. Es zeigen:
Fig. 1 eine perspektivische Ansicht einer ersten Ausführungsform eines erfindungsgemäßen Behandlungsinstruments in einem an ein Endoskop angebrachten Zustand in einer geöffneten Stellung;
Fig. 2 eine perspektivische Ansicht eines distalen Endes des in Fig. 1 gezeigten Behandlungsinstruments gemäß der ersten Ausführungsform in einer geschlossenen Stellung;
Fig. 3 eine Längsschnittansicht des in Fig. 2 gezeigten distalen Endes des Behandlungsinstruments gemäß der ersten Ausführungsform in der geschlossenen Stellung entsprechend der in Fig. 2 gezeigten Schnittebene III;
Fig. 4 eine Längsschnittansicht des in Fig. 1 gezeigten distalen Endes des Behandlungsinstruments gemäß der ersten Ausführungsform in der geöffneten Stellung entsprechend der in Fig. 1 gezeigten Schnittebene IV;
Fig. 5 eine Frontansicht des in Fig. 2 gezeigten Behandlungsinstruments gemäß der ersten Ausführungsform in der geschlossenen Stellung entsprechend dem in Fig. 2 gezeigten Pfeil V;
Fig. 6 eine Frontansicht des in Fig. 1 gezeigten Behandlungsinstruments gemäß der ersten Ausführungsform in der geöffneten Stellung entsprechend dem in Fig. 1 gezeigten Pfeil VI;
Fig. 7 eine Rückansicht des distalen Endes des Behandlungsinstruments gemäß der ersten Ausführungsform in der geschossenen Stellung;
Fig. 8 eine Rückansicht des distalen Endes des Behandlungsinstruments gemäß der ersten Ausführungsform in der geöffneten Stellung,
Fig. 9 eine perspektivische Ansicht eines erfindungsgemäßen Behandlungsinstruments gemäß einer zweiten Ausführungsform in einem an ein Endoskop angebrachten Zustand in einer geöffneten Stellung;
Fig. 10 eine der Fig. 4 entsprechende Längsschnittansicht eines erfindungsgemäßen Behandlungsinstruments gemäß einer dritten Ausführungsform;
Fig. 11 eine perspektivische Ansicht eines erfindungsgemäßen Behandlungsinstruments gemäß einer vierten Ausführungsform in einer geschlossenen Stellung;
Fig. 12 eine perspektivische Ansicht des in Fig. 11 gezeigten Behandlungsinstruments gemäß der vierten Ausführungsform in einer geöffneten Stellung;
Fig. 13 eine schematische Längsschnittansicht eines erfindungsgemäßen Behandlungsinstruments;
Fig. 14A und Fig. 14B schematische Querschnittsansichten zur Veranschaulichung unterschiedlicher Ausbildungen einer Stellmechanik gemäß der ersten und zweiten Ausführungsform;
Fig. 14C eine Fig. 14A und Fig. 14B entsprechende schematische Querschnittsansicht eines erfindungsgemäßen Behandlungsinstruments gemäß einer fünften Ausführungsform mit einer alternativen Ausbildung der Stellmechanik;
Fig. 15 eine Fig. 13 entsprechende schematische Längsschnittansicht eines erfindungsgemäßen Behandlungsinstruments gemäß einer sechsten Ausführungsform mit einer weiteren alternativen Ausbildung der Stellmechanik;
Fig. 16 eine Fig. 14A, Fig. 14B und Fig. 14C entsprechende schematische Querschnittsansicht des Behandlungsinstruments gemäß der sechsten Ausführungsform;
Fig. 17 eine Fig. 13 entsprechende schematische Längsschnittansicht eines erfindungsgemäßen Behandlungsinstruments;
Fig. 18A eine Fig. 14A, Fig. 14B und Fig. 14C entsprechende schematische Querschnittsansicht des Behandlungsinstruments gemäß der vierten Ausführungsform;
Fig. 18B eine Fig. 18A entsprechende schematische Querschnittsansicht eines erfindungsgemäßen Behandlungsinstruments gemäß einer siebten Ausführungsform;
Fig. 19 eine Fig. 17 entsprechende schematische Längsschnittansicht eines erfindungsgemäßen Behandlungsinstruments gemäß einer achten Ausführungsform;
Fig. 20 eine Fig. 18A und Fig. 18B entsprechende Querschnittsansicht des Behandlungsinstruments gemäß der achten Ausführungsform;
Fig. 21 eine Fig. 3 entsprechende Längsschnittansicht eines erfindungsgemäßen Behandlungsinstruments gemäß einer neunten Ausführungsform in einer geschlossenen Stellung;
Fig. 22 eine Fig. 4 entsprechende Längsschnittansicht des Behandlungsinstruments gemäß der neunten Ausführungsform in einer geöffneten Stellung;
Fig. 23 eine Fig. 3 entsprechende Längsschnittansicht eines erfindungsgemäßen Behandlungsinstruments gemäß einer zehnten Ausführungsform in einer geschlossenen Stellung;
Fig. 24 eine Fig. 4 entsprechende Längsschnittansicht des Behandlungsinstruments gemäß der zehnten Ausführungsform in einer geöffneten Stellung;
Fig. 25 eine Fig. 3 entsprechende Längsschnittansicht eines erfindungsgemäßen Behandlungsinstruments gemäß einer elften Ausführungsform in einer geschlossenen Stellung;
Fig. 26 eine Fig. 4 entsprechende Längsschnittansicht des Behandlungsinstruments gemäß der elften Ausführungsform in einer geöffneten Stellung;
Fig. 27 eine Fig. 5 entsprechende Frontansicht des Behandlungsinstruments gemäß der elften Ausführungsform in der geschlossenen Stellung;
Fig. 28 eine Fig. 7 entsprechende Rückansicht des Behandlungsinstruments gemäß der elften Ausführungsform in der geschlossenen Stellung;
Fig. 29 eine Fig. 3 entsprechende Längsschnittansicht eines erfindungsgemäßen Behandlungsinstruments gemäß einer zwölften Ausführungsform in einer geschlossenen Stellung; und
Fig. 30 eine Fig. 4 entsprechende Längsschnittansicht des Behandlungsinstruments gemäß der zwölften Ausführungsform in einer geöffneten Stellung.

Gleiche oder funktional äquivalente Merkmale sind in den einzelnen Figuren mit denselben Bezugszeichen versehen.

### Detaillierte Beschreibung bevorzugter Ausführungsformen

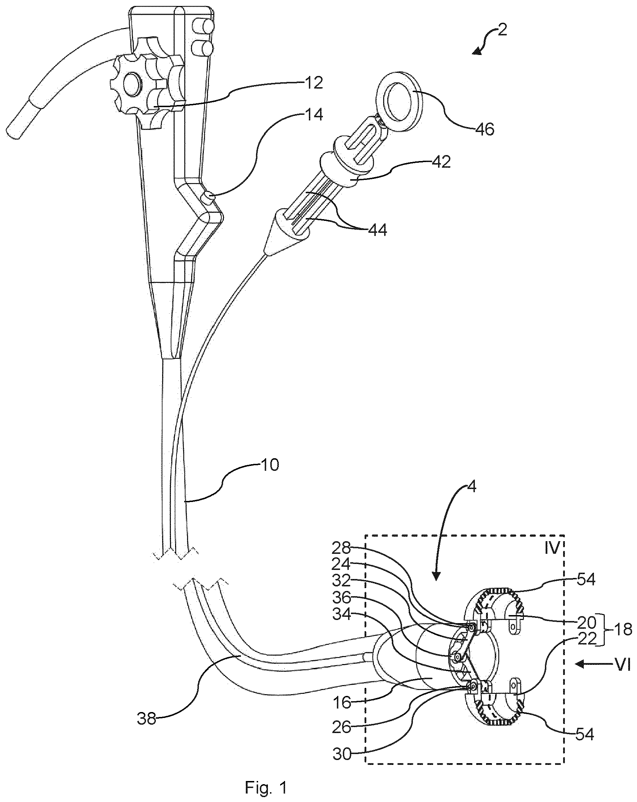

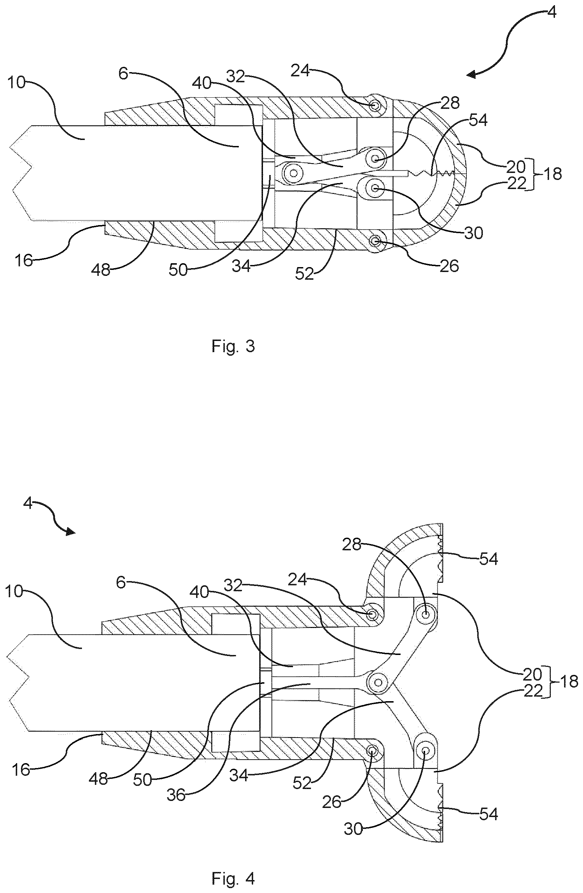

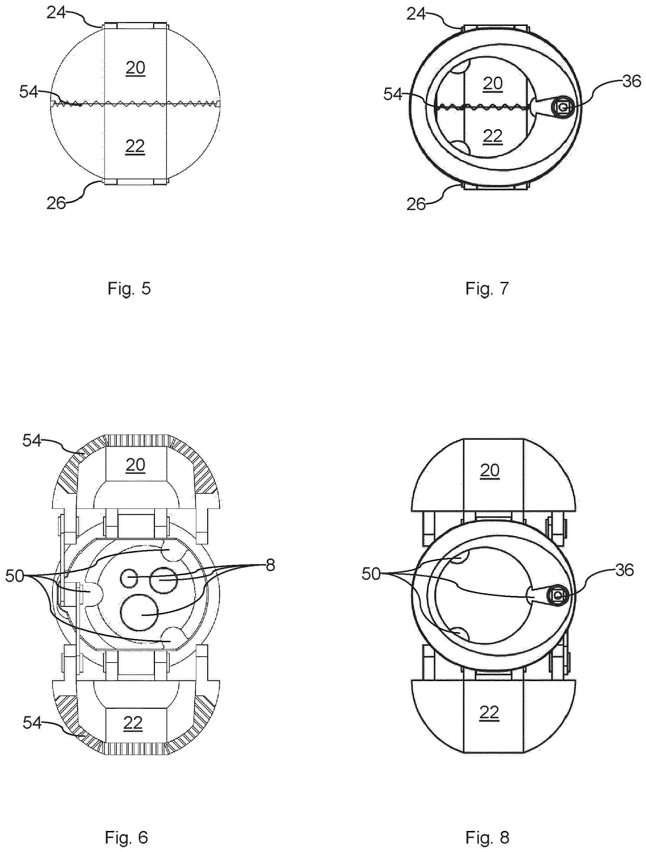

Grundsätzlich betrifft die Erfindung ein in Fig. 1 gezeigtes Behandlungsinstrument 4 zum Nachrüsten eines (handelsüblichen) Endoskops 2 der flexiblen Schaftbauart. Derartige Endoskope 2 sind dem Stand der Technik hinlänglich bekannt und werden deshalb im Folgenden nur oberflächlich beschrieben. So weist ein solches Endoskop 2 in der Regel einen Endoskopkopf 6 auf (siehe beispielsweise Fig. 3), in welchem diverse, im Folgenden nicht näher erläuterte, Funktionseinheiten 8, wie z.B. ein Ausgang eines primären bzw. endoskopinternen Arbeitskanals, eine Optik zur Bildgebung, Leuchtmittel und/oder eine Spülkanalaustrittsöffnung eines Spülkanals, angeordnet sind (siehe Fig. 6). Weiter weist ein solches Endoskop 2 zumeist einen flexiblen Schaft 10 mit passiv biegesteifen Abschnitten und vorzugsweise einem aktiv abkrümmbaren Abschnitt (Deflecting) sowie einen Handgriff 12 mit diversen Bedienelementen und Anschlüssen zum Anschließen der diversen Funktionseinheiten 8 an eine Basisstation oder dergleichen auf. Insbesondere ist an dem Handgriff 12 ein Eingang 14 des primären Arbeitskanals vorgesehen.

Das Behandlungsinstrument 4 weist einen Hohlkörper 16 auf, an dessen distalen Ende ein Greiforgan 18 mit zwei sich gegenüberliegenden Greifteilen 20 und 22 vorgesehen ist. Die beiden Greifteile 20 und 22 sind jeweils über ein Gelenk 24 bzw. 26 mit einem entsprechenden Abschnitt an einem distalen Rand des Hohlkörpers 16 schwenkbeweglich verbunden.

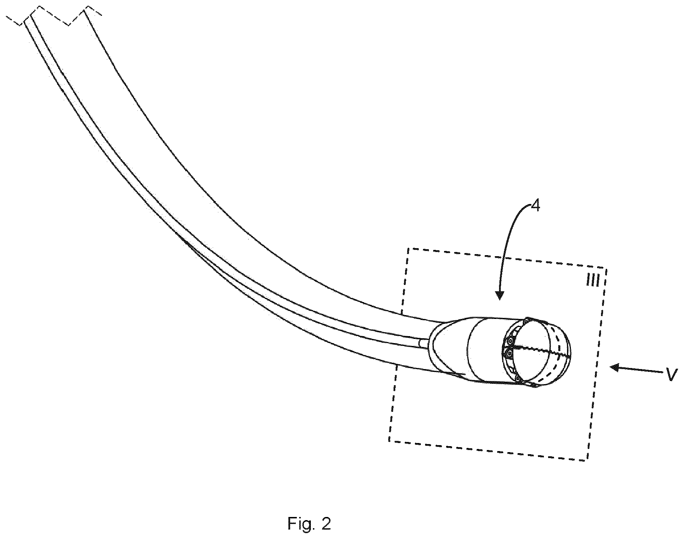

Um von einer in Fig.1 gezeigten offenen bzw. geöffneten Stellung in eine in Fig. 2 gezeigte geschlossene Stellung verstellbar zu sein, sind die beiden Greifteile 20 und 22 jeweils über ein Gelenk 28 bzw. 30 mit einem entsprechenden Pleuel 32 bzw. 34 verbunden. Die beiden Pleuel 32 bzw. 34 sind wiederum mit einem distalen Ende eines Stellglieds 36 verbunden. Vorzugsweise ist das Stellglied 36 derart ausgebildet, dass ein biegesteifes Element das distale Ende des Stellglieds 36 bildet und das biegesteife Element mit einem Zug-Schub-Draht verbunden ist, welcher sich von dem biegesteifen Element aus in proximaler Richtung erstreckt. Bei einer solchen Ausbildung bilden demnach das biegesteife Element und der Zug-Schub-Draht zusammen das Stellglied 36.

Von einem proximalen Ende des Hohlkörpers 16 aus erstreckt sich in proximaler Richtung ein Schlauch 38. Anstelle dieses Schlauchs 38 kann alternativ eine flexible Drahtspirale mit Ummantelung vorgesehen sein. Von einem distalen Ende des Hohlkörpers 16 aus durch eine Außenwand des Hohlkörpers 16 bis zum proximalen Ende des Hohlkörpers 16 sowie von dem proximalen Ende des Hohlkörpers 16 durch den Schlauch 38 bis zu einem proximalen Ende des Schlauches 38 erstreckt sich ein sekundärer bzw. endoskopexterner Arbeitskanal 40. In diesem sekundären Arbeitskanal 40 ist das Stellglied 36 bzw. sind das biegesteife Element sowie der Zug-Schub-Draht des Stellglieds 36 verschiebbar gelagert.

Um das Stellglied 36 einfach verschieben zu können, ist an dem proximalen Ende des Stellglieds 36 bzw. an dem proximalen Ende des Zug-Schub-Drahts ein Schieber 42 vorgesehen, welcher mit zwei zueinander parallel verlaufenden sich vom proximalen Ende des Schlauches 38 aus in proximaler Richtung erstreckenden Führungsschienen 44 verschiebbar verbunden ist. An proximalen Enden der Führungsschienen 44 ist ein Ring 46 derart vorgesehen, dass die Enden der Führungsschienen 44 miteinander verbunden sind und der Schieber 42 somit in den Führungsschienen 44 gefangen ist. Um das Stellglied 36 zu verschieben, kann eine das Behandlungsinstrument 4 verwendende Person den Daumen einer ihrer Hände in den Ring 46 stecken, kann den Schieber 42 zwischen Zeige- und Mittelfinger derselben Hand einklemmen und kann den Schieber 42 mittels Bewegen des Zeigefingers und des Mittelfingers relativ zum Daumen verstellen.

Um mit dem Endoskopkopf 6 verbindbar zu sein, weist der Hohlkörper 16 an einem proximalen Ende eine im Wesentlichen kreiszylindrische Ausnehmung 48 auf, in welche der Endoskopkopf mit Presspassung hineinsteckbar ist (siehe Fig. 3 und Fig. 4). Alternativ oder zusätzlich dazu könnten für eine Verbindung mit einem Endoskopkopf auch Klipverbindungsmittel an dem Hohlkörper 16 vorgesehen sein.

An einem distalen Ende der Ausnehmung 48 sind drei über den Innenumfang des Hohlkörpers 16 gleichmäßig verteilte Anschläge 50 vorgesehen, welche die maximale Eintauchtiefe des Endoskopkopfs 6 begrenzen. Alternativ können die Anschläge 50 über den Innenumfang des Hohlkörpers 16 in ungleichmäßiger Art verteilt sein.

Distal von den Anschlägen 50 weist der Hohlkörper 16 eine im Wesentlichen kreiszylindrische sich bis zum distalen Ende des Hohlkörpers 16 erstreckende Ausnehmung 52 auf, deren Innendurchmesser größer ist als ein Innendurchmesser der Ausnehmung 48 am proximalen Ende des Hohlkörpers 16. Alternativ ist es auch möglich, dass die Innendurchmesser der Ausnehmungen 48 und 52 gleich groß sind oder dass der Innendurchmesser der Ausnehmung 48 größer ist als der Innendurchmesser der Ausnehmung 52.

Die beiden Greifteile 20 und 22 sind derart schalen- bzw. schaufelförmig ausgebildet, dass sie in der geschlossenen Stellung, wie in Fig. 3, Fig. 5 und Fig. 7. gezeigt, zusammen eine haubenförmige Struktur bilden können, welche den Hohlkörper 16 in distaler Richtung vollständig verschließt. Wird das erfindungsgemäße Behandlungsinstrument 4 auf einem Endoskopkopf 6 angebracht, ist es somit möglich, den Endoskopkopf 6 bzw. ein Objektiv einer Optik des Endoskops 6 mit den Greifteilen 20 und 22 in der geschlossenen Stellung vor Verunreinigungen abzuschirmen.

Die beiden Greifteile 20 und 22 weisen jeweils eine Greifleiste 54 auf. Die beiden Greifleisten 54 weisen jeweils Vorsprünge und Rücksprünge auf. Die Greifleisten 54 sind derart komplementär ausgebildet, dass in der geschlossenen Stellung der Greifteile 20 und 22 die Vorsprünge der Greifleiste 54 des Greifteils 20 in die Rücksprünge der Greifleiste 54 des Greifteils 22 eintauchen können und die Vorsprünge der Greifleiste 54 des Greifteils 22 in die Rücksprünge der Greifleiste 54 des Greifteils 20 eintauchen können. Alternativ können die Greifleisten 54 auch glatt ausgebildet sein und derart aufeinander abgestimmt sein, dass die Greifleisten 54 in der geschlossenen Stellung der Greifteile 20 und 22 bündig miteinander in Kontakt stehen.

Die Gelenke 24 und 26, welche die Greifteile 20 und 22 mit dem Hohlkörper 16 verbinden, sind derart ausgebildet, dass sich deren Schwenkachsen tangential zu einem Umfang des Hohlkörpers 16 erstrecken. Somit ist es möglich, die Greifteile 20 und 22 derart nach außen zu klappen, dass sie in der geöffneten Stellung, wie in Fig. 4, Fig. 6 und Fig. 8 gezeigt, eine distale Seite der Ausnehmung 52 vollständig freigeben.

Die in den Figuren 1 bis 8 gezeigte erste Ausführungsform des erfindungsgemäßen Behandlungsinstruments 4 weist lediglich ein Stellglied 36 und entsprechend lediglich einen endoskopexternen Arbeitskanal 40 auf.

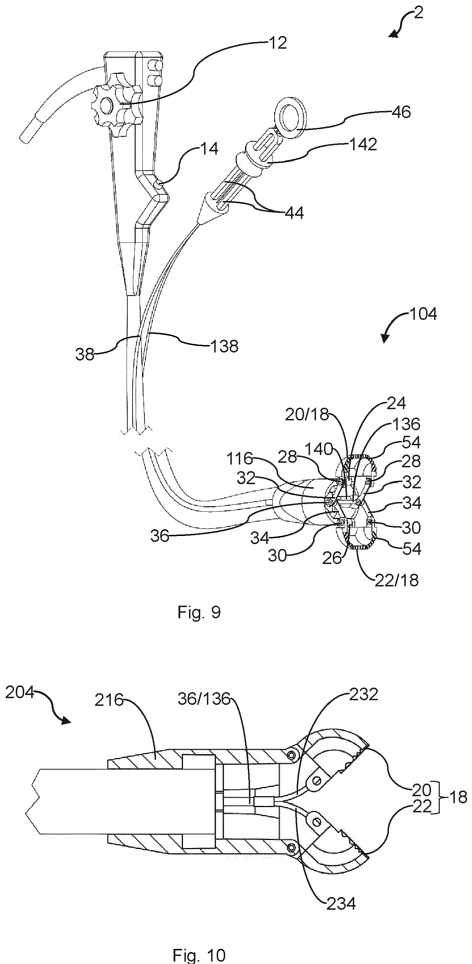

Um ein im Vergleich zum Behandlungsinstrument 4 gemäß der ersten Ausführungsform stärkeres Greifen zu ermöglichen, kann entsprechend einer zweiten Ausführungsform, wie in Fig. 9 gezeigt, ein erfindungsgemäßes Behandlungsinstrument 104 zusätzlich mit einem zweiten Stellglied 136, einem zweiten Schlauch 138 und einem zweiten endoskopexternen Arbeitskanal 140 ausgestattet sein. Das zweite Stellglied 136 ist entsprechend dem (ersten) Stellglied 36 über die Pleuel 32 und 34 mit den Greifteilen 20 und 22 verbunden. Die Pleuel 32 und 34 des zweiten Stellglieds 136 sind über Gelenke 28 und 30 mit den Greifteilen 20 und 22 schwenkbeweglich verbunden. Das zweite Stellglied 136 bzw. dessen distales Ende, die Pleuel 32 und 34 sowie die Gelenke 28 und 30 des zweiten Stellglieds 136 und der zweite Arbeitskanal 140 bzw. dessen distales Ende sind an einer Seite des Hohlkörpers 116 angeordnet, welche einer Seite des Hohlkörpers 116 gegenüberliegt, an der das (erste) Stellglied 36 bzw. dessen distales Ende, die Pleuel 32 und 34 sowie die Gelenke 28 und 30 des (ersten) Stellglieds 36 und der (erste) Arbeitskanal 40 bzw. dessen distales Ende angeordnet sind.

Der (erste) Schlauch 38 und der zweite Schlauch 138 sind an ihren proximalen Enden miteinander verbunden. Das (erste) Stellglied 36 und das zweite Stellglied 136 sind an ihren proximalen Enden miteinander verbunden und sind entsprechend der ersten Ausführungsform über einen Schieber 142 betätigbar.

Gemäß den ersten beiden Ausführungsformen sind die Greifteile 20 und 22 über biegesteife Pleuel 32 und 34 mit dem entsprechenden Stellglied 36 bzw. 136 verbunden. Alternativ dazu kann gemäß einer dritten Ausführungsform, wie in Fig. 10 gezeigt, die Verbindung zwischen dem Stellglied 36 bzw. 136 und den Greifteilen 20 bzw. 22 mittels flexibler Elemente 232 bzw. 234 bewerkstelligt sein. Diese flexiblen Elemente 232 bzw. 234 können fest bzw. biegefest mit dem Stellglied 36 bzw. 136 und/oder fest bzw. biegefest mit dem entsprechenden Greifteil 20 bzw. 22 verbunden sein, so dass die Gelenke 28 und 30 eingespart werden können.

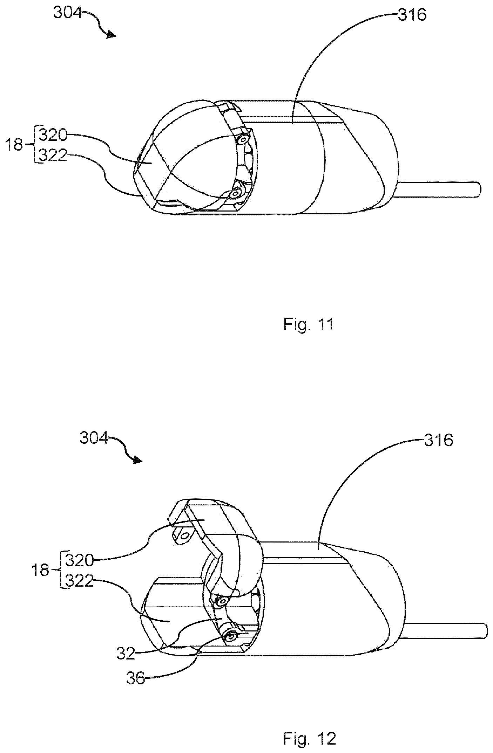

Die erfindungsgemäßen Behandlungsinstrumente 4, 104 und 204 gemäß der ersten, zweiten und dritten Ausführungsform weisen jeweils zwei Greifteile 20 und 22 auf, welche beide schwenkbeweglich mit einem Hohlkörper 16, 116 bzw. 216 verbunden sind. Gemäß einer vierten Ausführungsform kann ein erfindungsgemäßes Behandlungsinstrument 304 auch zwei Greifteile 320 und 322 aufweisen, von denen nur eines (das Greifteil 320) schwenkbeweglich mit einem Hohlkörper 316 verbunden ist, wohingegen das andere (das Greifteil 322) fest bzw. biegefest mit dem Hohlkörper 316 verbunden ist. Wie bei den Behandlungsinstrumenten 4, 104, und 204 gemäß der ersten, zweiten und dritten Ausführungsform bilden die beiden Greifteile 320 und 322 zusammen eine haubenförmige Struktur, welche eine distale Seite des Hohlkörpers 316 verschließt, wenn die Greifteile 320 und 322 in einer geschlossenen Stellung sind (siehe Fig. 11). Im Gegensatz zu den Greifteilen 20 und 22, welche identisch zueinander ausgebildet sind, sind die Greifteile 320 und 322 unterschiedlich zueinander ausgeformt. Das fest mit dem Hohlkörper verbundene Greifteil 322 ist im Wesentlichen ein in distaler Richtung vorragender Vorsprung bzw. eine in distaler Richtung vorragende rinnenförmige Zunge. Lediglich das schwenkbeweglich mit dem Hohlkörper 316 verbundene Greifteil 320 ist schalen- bzw. schaufelförmig ausgebildet. Die Stellmechanik zum Verstellen des Greifteils 320 entspricht der Stellmechanik zum Verstellen der Greifteile 20 und 22.

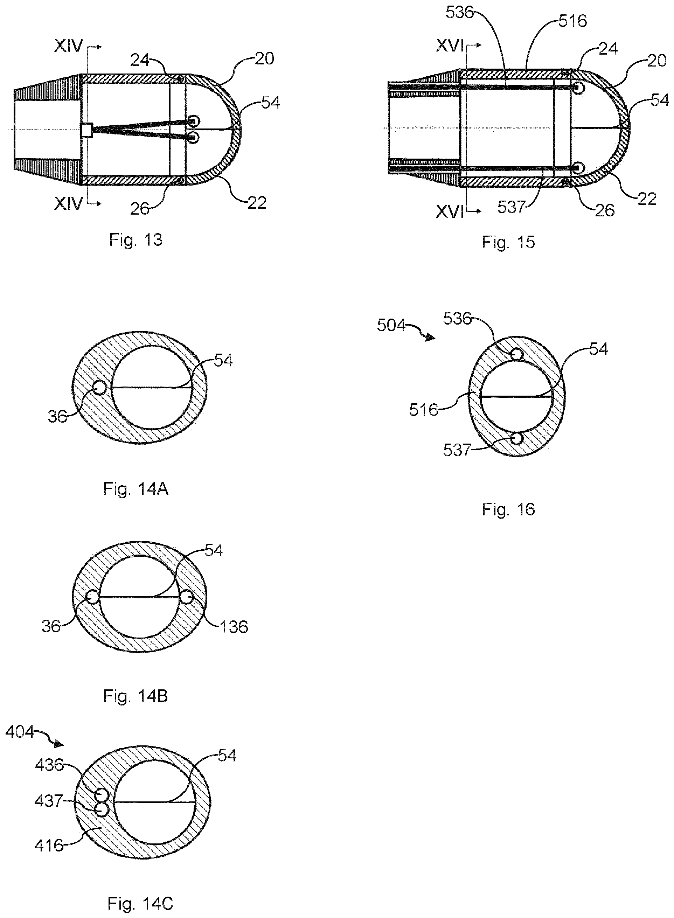

Wie ein Vergleich der ersten mit der zweiten Ausführungsform zeigt, kann ein erfindungsgemäßes Behandlungsinstrument ein oder zwei Stellglieder aufweisen. Die Figuren 14A und 14B, welche entsprechend der ersten und der zweiten Ausführungsform zwei alternative schematische Querschnittsansichten entlang der in Fig. 13 gezeigten Linie XIV-XIV zeigen, veranschaulichen die Anordnung der Stellglieder 36 und 136. Gemäß der ersten Ausführungsform kann ein einziges Stellglied 36 derart in dem Hohlkörper 16 verschieblich gelagert sein, dass sich das distale Ende des Stellglieds 36 zusammen mit den Greifleisten 54 in einer gemeinsamen Ebene erstreckt, wenn die Greifteile 20 und 22 in der geschlossenen Stellung sind. Gemäß der zweiten Ausführungsform können zwei Stellglieder 36 und 136 derart in dem Hohlkörper 116 verschieblich gelagert sein, dass sich die distalen Enden der beiden Stellglieder 36 und 136 zusammen mit den Greifleisten 54 in einer gemeinsamen Ebene erstrecken, wenn die Greifteile 20 und 22 in der geschlossenen Stellung sind. Um dies zu ermöglichen, sind die beiden Stellglieder 36 und 136 gemäß der zweiten Ausführungsform bzw. deren distale Enden an gegenüberliegenden Seiten des Hohlkörpers 116 angeordnet.

Gemäß einer fünften und sechsten Ausführungsform ist es jedoch auch möglich, ein erfindungsgemäßes Behandlungsinstrument 404 bzw. 504 mit zwei Stellgliedern 436 und 437 bzw. 536 und 537 auszustatten, welche sich nicht zusammen mit den Greifleisten 54 in einer gemeinsamen Ebene erstrecken bzw. deren distale Enden sich nicht zusammen mit den Greifleisten 54 in einer gemeinsamen Ebene erstrecken, wenn die Greifteile 20 und 22 in der geschlossenen Stellung sind.

Wie Fig. 14C zeigt, ist das Behandlungsinstrument 404 gemäß der fünften Ausführungsform ausgestattet mit einem Stellglied 436, welches lediglich mit dem Greifteil 20 verbunden ist, und mit einem Stellglied 437, welches lediglich mit dem Greifteil 22 verbunden ist. Die beiden Stellglieder 436 und 437 bzw. deren distale Enden sind an einer gemeinsamen Seite des Hohlkörpers 416 derart angeordnet, dass sie in der geschlossenen Stellung der Greifteile 20 und 22 unmittelbar zu einer von den Greifleisten 54 aufgespannten Ebene benachbart sind.

Wie Fig. 15 und Fig. 16 (eine Querschnittsansicht entlang der in Fig. 15 gezeigten Linie XVI-XVI) zeigen, ist das Behandlungsinstrument 504 gemäß der sechsten Ausführungsform ausgestattet mit einem Stellglied 536, welches lediglich mit dem Greifteil 20 verbunden ist, und mit einem Stellglied 537, welches lediglich mit dem Greifteil 22 verbunden ist. Die beiden Stellglieder 536 und 537 bzw. deren distale Enden sind an gegenüberliegenden Seiten des Hohlkörpers 516 derart angeordnet, dass sie sich in einer gemeinsamen Ebene erstrecken, welche senkrecht zu den Schwenkachsen der Gelenke 24 und 26 verläuft.

Durch das Vorsehen zweier Stellglieder 436 und 437 bzw. 536 und 537, welche jeweils nur mit einem Greifteil 20 bzw. 22 verbunden sind, kann die Ansteuerung der der Greifteile 20 bzw. 22 verfeinert werden.

Durch die Anordnung der Stellglieder 536 und 537 gemäß der sechsten Ausführungsform ist es möglich, die Stellglieder 536 und 537 ohne Zwischenteile (wie Pleuel, Schubstangen oder flexible Elemente) mit dem entsprechenden Greifteil zu verbinden.

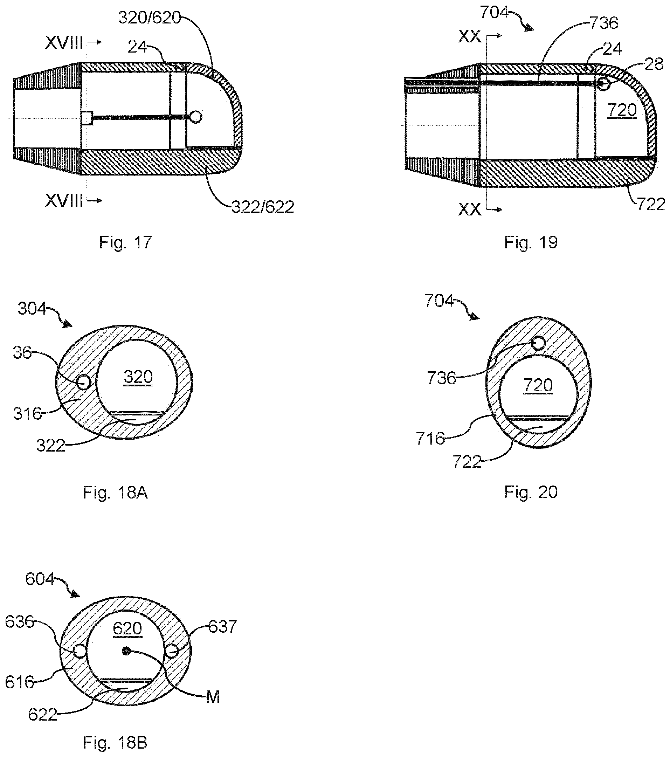

Die Figuren 17, 18A, 18B, 19 und 20 zeigen unterschiedliche Variationen der Anordnung eines Stellglieds oder mehrerer Stellglieder bzw. der Anordnung eines distalen Endes eines Stellglieds oder mehrerer distaler Enden von mehreren Stellgliedern bei einem erfindungsgemäßen Behandlungsinstrument mit nur einem schwenkbeweglich mit dem Hohlkörper verbundenen Greifteil.

Fig. 18A ist eine schematische Querschnittsansicht entlang der in Fig. 17 gezeigten Linie XVIII-XVIII und veranschaulicht die Anordnung des Stellglieds 36 bei dem Behandlungsinstrument 304 gemäß der in den Figuren 11 und 12 gezeigten vierten Ausführungsform. Fig. 18B ist eine schematische Querschnittsansicht entlang der in Fig. 17 gezeigten Linie XVIII-XVIII und veranschaulicht ein Behandlungsinstrument 604 gemäß einer siebten Ausführungsform mit einem Stellglied 636 und einem zweiten Stellglied 637. Die beiden Stellglieder 636 und 637 bzw. die distalen Enden der beiden Stellglieder 636 und 637 sind an zwei sich gegenüberliegenden Seiten des Hohlkörpers 616 angeordnet. Die beiden Stellglieder 636 und 637 bzw. die distalen Enden der beiden Stellglieder 636 und 637 liegen in einer gemeinsamen Ebene, welche sich durch eine Längsmittelachse M des Hohlkörpers 616 erstreckt.

Wie Fig. 19 und Fig. 20 (eine Querschnittsansicht entlang der in Fig. 19 gezeigten Linie XX-XX) zeigen, kann gemäß einer achten Ausführungsform bei einem Behandlungsinstrument 704 mit lediglich einem mit einem Hohlkörper 716 schwenkbeweglich verbundenen Greifteil 720 ein Stellglied 736 derart angeordnet sein, dass ein Gelenk 28 zwischen dem Stellglied 736 und dem Greifteil 720 unmittelbar zu einem Gelenk 24 zwischen dem Greifteil 720 und dem Hohlkörper 716 benachbart ist. Wie bei dem Behandlungsinstrument 504 gemäß der sechsten Ausführungsform kann durch diese Anordnung des Stellglieds 736 eine unmittelbare Verbindung zwischen dem Stellglied 736 und dem Greifteil 720 ohne Zwischenteile (wie Pleuel, Schubstangen oder flexible Elemente) ermöglicht werden.

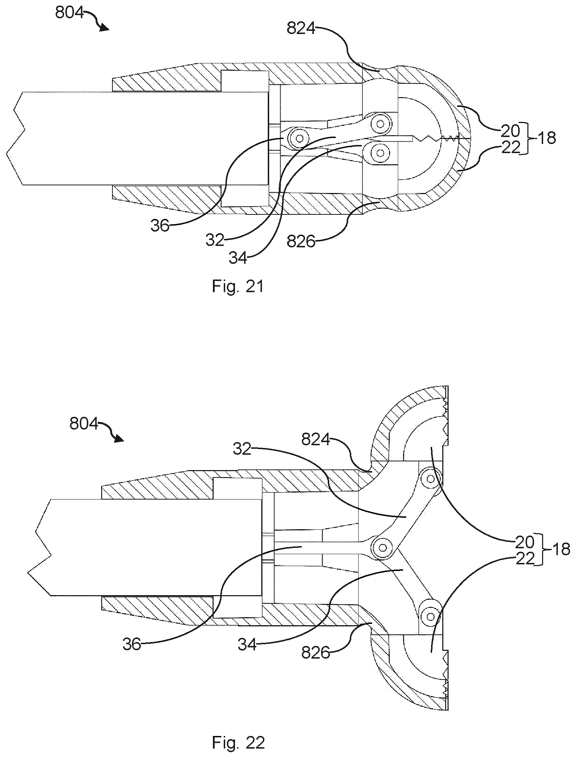

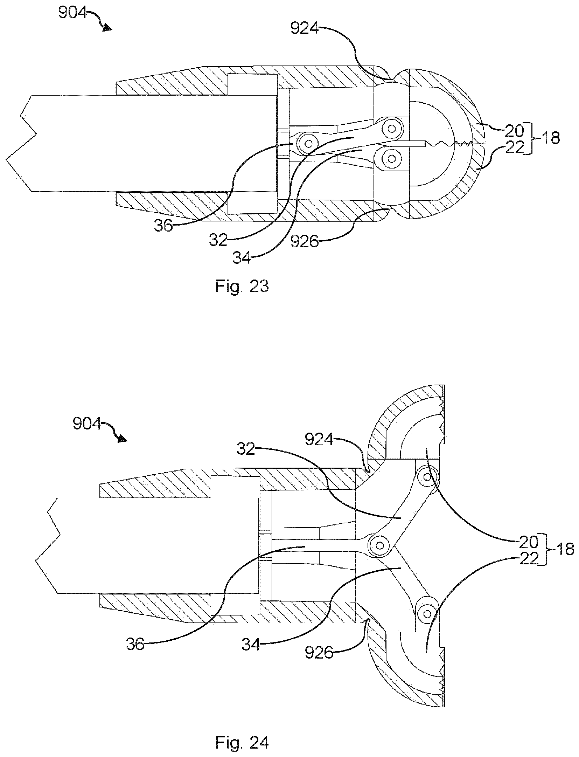

Fig. 21 und Fig. 22 zeigen ein Behandlungsinstrument 804 gemäß einer neunten Ausführungsform. Fig. 23 und Fig. 24 zeigen ein Behandlungsinstrument 904 gemäß einer zehnten Ausführungsform.

Das Behandlungsinstrument 804 gemäß der neunten Ausführungsform unterscheidet sich von dem Behandlungsinstrument 4 gemäß der ersten Ausführungsform lediglich insofern, als die Greifteile 20 und 22 nicht über Drehgelenke 24 und 26, sondern über Festkörpergelenke 824 und 826 mit dem Hohlkörper 16 verbunden sind.

Das Behandlungsinstrument 904 gemäß der zehnten Ausführungsform unterscheidet sich von dem Behandlungsinstrument 4 gemäß der ersten Ausführungsform lediglich insofern, als die Greifteile 20 und 22 nicht über Drehgelenke 24 und 26, sondern über Filmscharniere 924 und 926 mit dem Hohlkörper 16 verbunden sind.

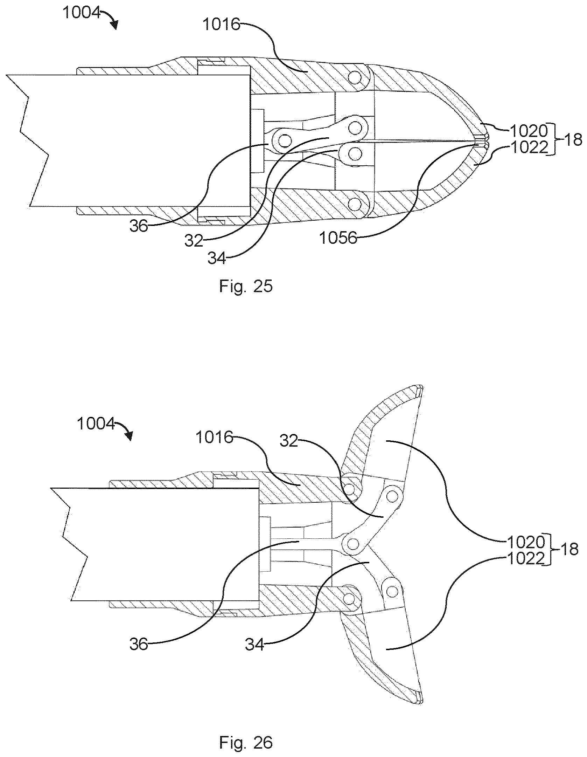

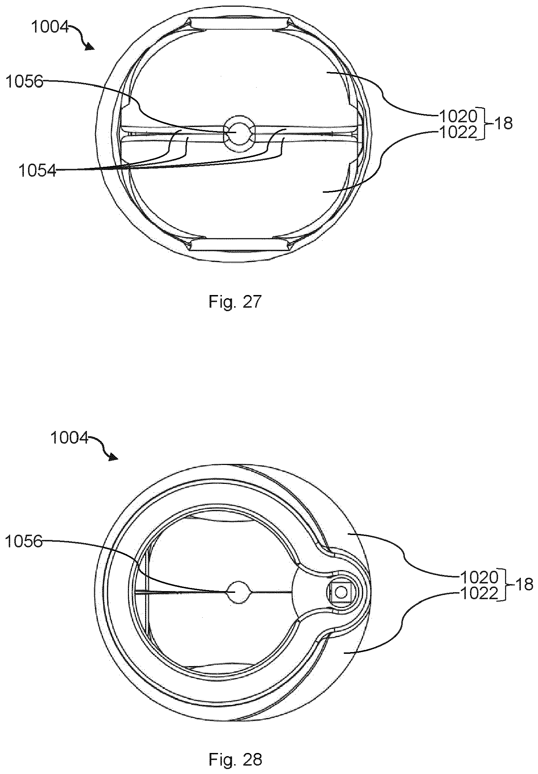

Fig. 25, Fig. 26, Fig. 27 und Fig. 28 zeigen ein Behandlungsinstrument 1004 gemäß einer elften Ausführungsform. Das Behandlungsinstrument 1004 gemäß der elften Ausführungsform unterscheidet sich von dem Behandlungsinstrument 4 gemäß der ersten Ausführungsform hinsichtlich zweier Aspekte.

Zum einen sind die Greifteile 1020 und 1020 des Behandlungsinstruments 1004 derart ausgebildet, dass sie in einer geschlossenen Stellung (wie in Fig. 25 gezeigt) zusammen eine haubenförmige Struktur bilden können, welche nicht nur den Hohlkörper 1016 in distaler Richtung verschließt, sondern gleichzeitig auch eine konisch spitz zulaufende Form bildet. Anders ausgedrückt sind die Greifteile 1020 und 1022 derart ausgebildet, dass sie in der geschlossenen Stellung einen sich in proximale Richtung konisch aufweitenden Abschnitt aufweisen bzw. bilden. Zusätzlich zur Abschirmung vor Verunreinigungen ist es mit dieser geometrischen Form möglich, Strikturen gegebenenfalls aufzuweiten und zu passieren. Neben der passiven Aufweitung durch die konische Form, können die Greifteile 1020 und 1022 aktiv durch Öffnen Druck auf die Striktur ausüben und den Aufweitungsprozess unterstützen. In einem distalen Abschnitt weist auch der Hohlkörper 1016 einen sich in proximale Richtung konisch aufweitenden Abschnitt auf.

Zum anderen unterscheiden sich die Greifteile 1020 und 1022 gemäß der zwölften Ausführungsform von den Greifteilen 20 und 22 gemäß der ersten Ausführungsform bezüglich einer Aussparung 1056. Die Aussparung 1056 ist teilweise in dem Greifteil 1020 und teilweise in dem Greifteil 1022 ausgebildet. Genauer gesagt sind Greifleisten 1054 der Greifteile 1020 und 1022 jeweils mittig durch einen rinnenförmigen Abschnitt unterbrochen. Befinden sich die Greifteile 1020 und 1022 in der geschlossenen Stellung, liegen die rinnenförmigen Abschnitte der beiden Greifteile 1020 und 1022 aneinander an und bilden zusammen die Aussparung 1056. Die Aussparung weist einen kreisrunden Umriss auf. Die Öffnungsweite bzw. der Durchmesser der Aussparung 1056 beträgt 1,1 mm. Die Aussparung 1056 ermöglicht es, einen Führungsdraht durch das geschlossene Greiforgan zu stecken, ohne den Schutz eines Objektivs einer Optik eines Endoskops übermäßig aufzugeben. Insbesondere ist die Aussparung 1056 derart angeordnet und ist ein entsprechendes Endoskop 2 derart ausgebildet, dass das Greiforgan 18 in der geschlossenen Stellung lediglich einen Führungskanal für den Führungsdraht in distaler Richtung freigibt und alle übrigen Funktionseinheiten 8, wie z. B. einen Ausgang eines primären bzw. endoskopinternen Arbeitskanals, eine Optik zur Bildgebung, Leuchtmittel und/oder eine Spülkanalaustrittsöffnung eines Spülkanals, in distaler Richtung vollständig verdeckt.

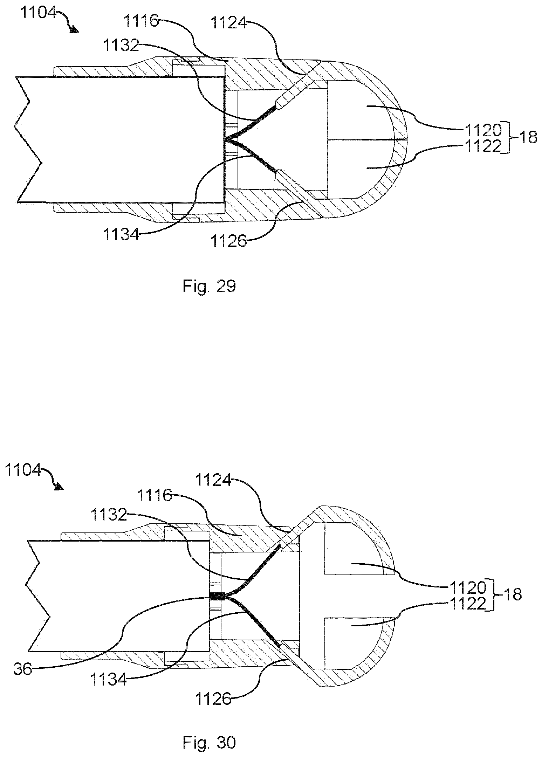

Fig. 29 und Fig. 30 zeigen ein Behandlungsinstrument 1104 gemäß einer zwölften Ausführungsform. Das Behandlungsinstrument 1104 gemäß der zwölften Ausführungsform unterscheidet sich von dem Behandlungsinstrument 4 gemäß der ersten Ausführungsform insofern, als die Greifteile 1120 und 1122 nicht über Drehgelenke 24 und 26, sondern über Schubgelenke 1124 und 1126 mit einem Hohlkörper 1116 verbunden sind. Die Schubgelenke 1124 und 1126 sind derart ausgebildet, dass die Greifteile 1120 und 1122 sowohl in distale Richtung als auch in eine radiale Richtung nach außen bewegt werden, wenn die Greifteile 1124 und 1126 über fest mit den Greifteilen 1124 und 1126 verbundene flexible Elementen 1132 und 1134 in eine geöffnete Stellung (siehe Fig. 30) gebracht werden, und dass die Greifteile 1120 und 1122 sowohl in proximale Richtung als auch in eine radiale Richtung nach innen bewegt werden, wenn die Greifteile 1124 und 1126 über die flexiblen Elementen 1132 und 1134 in eine geschlossene Stellung (siehe Fig. 29) gebracht werden.

Anders ausgedrückt ist das Greiforgan 18 bei dem Behandlungsinstrument 1104 gemäß der zwölften Ausführungsform als Parallelgreifer ausgebildet.

### Bezugszeichenliste

- 2: Endoskop
- 4: Behandlungsinstrument gemäß erster Ausführungsform
- 6: Endoskopkopf
- 8: Funktionseinheit im Endoskopkopf
- 10: Schaft des Endoskops
- 12: Handgriff des Endoskops
- 14: Eingang des endoskopinternen Arbeitskanal
- 16: Hohlkörper
- 18: Greiforgan
- 20, 22: Greifteil
- 24, 26: Gelenk zwischen Hohlkörper und Greifteil
- 28, 30: Gelenk zwischen Greifteil und Pleuel
- 32, 34: Pleuel
- 36: Stellglied
- 38: Schlauch
- 40: Endoskopexterner Arbeitskanal
- 42: Schieber
- 44: Führungsschiene
- 46: Ring an Führungsschienen
- 48: Ausnehmung am proximalen Ende des Hohlkörpers
- 50: Anschlag
- 52: Ausnehmung am distalen Ende des Hohlkörpers
- 54: Greifleiste

- 104: Behandlungsinstrument gemäß zweiter Ausführungsform
- 116: Hohlkörper gemäß zweiter Ausführungsform
- 136: Zweites Stellglied
- 138: Zweiter Schlauch
- 140: Zweiter endoskopexterner Arbeitskanal
- 142: Schieber gemäß zweiter Ausführungsform
- 204: Behandlungsinstrument gemäß dritter Ausführungsform
- 216: Hohlkörper gemäß dritter Ausführungsform
- 232, 234: Flexibles Element

- 304: Behandlungsinstrument gemäß vierter Ausführungsform
- 316: Hohlkörper gemäß vierter Ausführungsform
- 320: Schwenkbewegliches Greifteil
- 322: Festes Greifteil

- 404: Behandlungsinstrument gemäß fünfter Ausführungsform
- 416: Hohlkörper gemäß fünfter Ausführungsform
- 436, 437: Stellglied gemäß fünfter Ausführungsform

- 504: Behandlungsinstrument gemäß sechster Ausführungsform
- 516: Hohlkörper gemäß sechster Ausführungsform
- 536, 537: Stellglied gemäß sechster Ausführungsform

- 604: Behandlungsinstrument gemäß siebter Ausführungsform
- 616: Hohlkörper gemäß siebter Ausführungsform
- 620: Schwenkbewegliches Greifteil gemäß siebter Ausführungsform
- 622: Festes Greifteil gemäß siebter Ausführungsform
- 636, 637: Stellglied gemäß siebter Ausführungsform

- 704: Behandlungsinstrument gemäß achter Ausführungsform
- 716: Hohlkörper gemäß achter Ausführungsform
- 720: Schwenkbewegliches Greifteil gemäß achter Ausführungsform
- 722: Festes Greifteil gemäß achter Ausführungsform
- 736: Stellglied gemäß achter Ausführungsform

- 804: Behandlungsinstrument gemäß neunter Ausführungsform
- 824, 826: Festkörpergelenk
- 904: Behandlungsinstrument gemäß zehnter Ausführungsform
- 924, 926: Filmscharnier

- 1004: Behandlungsinstrument gemäß elfter Ausführungsform
- 1016: Hohlkörper gemäß elfter Ausführungsform
- 1020, 1022: Greifteil gemäß elfter Ausführungsform
- 1054: Greifleiste gemäß elfter Ausführungsform
- 1056: Aussparung in Greifteil

- 1104: Behandlungsinstrument gemäß zwölfter Ausführungsform
- 1116: Hohlkörper gemäß zwölfter Ausführungsform
- 1120, 1122: Greifteil gemäß zwölfter Ausführungsform
- 1124, 1126: Schubgelenk
- 1132, 1134: Flexibles Element gemäß zwölfter Ausführungsform

## Patentansprüche

1. Behandlungsinstrument (4; 104; 204; 304; 404; 504; 604; 704; 804; 904; 1004; 1104), welches dafür vorgesehen und ausgebildet ist, mit einem distalen Ende oder Endabschnitt eines Endoskops (2) gekoppelt zu werden und welches folgende Elemente hat:
- einen im Wesentlichen hülsenförmigen Hohlkörper (16; 116; 216; 316; 416; 516; 616; 716; 1016; 1116), dessen Umfangswand innenseitig einen axial sich erstreckenden Hohlraum umgibt sowie einen proximalen Kopplungsabschnitt bildet, der dafür vorgesehen und ausgebildet ist, an/auf einen Endoskopschaft oder Endoskopkopf angesetzt/aufgesteckt zu werden,
- ein Greiforgan (18), das an einem distalen Umfangswandabschnitt des Hohlkörpers (16; 116; 216; 316; 416; 516; 616; 716; 1016; 1116) gelagert oder angelenkt ist und
- eine zur Betätigung des Greiforgans (18) zumindest aus einer Freigabeposition in eine Greifposition vorgesehene Stellmechanik, welche einen proximalen Abschnitt hat, der sich von dem Hohlkörper (16; 116; 216; 316; 416; 516; 616; 716; 1016; 1116) aus in eine proximale Richtung erstreckt sowie einen distalen Abschnitt hat, der in dem Hohlraum aufgenommen ist und ein distales Ende aufweist, das mit dem Greiforgan (18) an dessen dem Hohlraum zugewandter Innenseite gekoppelt ist, wobei
- das Greiforgan (18) in Greifposition die Form einer Kappe; Klappe, Kuppel, Tür oder eines Dachs ausbildet, mit welcher der Hohlraum an einem distalen Ende des Hohlkörpers (16; 116; 216; 316; 416; 516; 616; 716; 1016; 1116) zumindest mit Erreichen der Greifposition vollständig verschlossen ist und das Greiforgan (18) in Freigabeposition eine Stirnfläche des Hohlkörpers (16; 116; 216; 316; 416; 516; 616; 716; 1016; 1116) vollständig in distaler Richtung freigibt.

2. Behandlungsinstrument (4; 104; 204; 304; 404; 504; 604; 704; 804; 904; 1004; 1104) nach Anspruch 1, **dadurch gekennzeichnet, dass** das Greiforgan (18) zumindest abschnittsweise transparent ausgebildet ist.

3. Behandlungsinstrument (4; 104; 204; 304; 404; 504; 604; 704; 804; 904; 1004; 1104) nach Anspruch 1 oder 2, **dadurch gekennzeichnet, dass** das Greiforgan (18) an einer Stirnfläche einen flachen Plattenabschnitt, einen einfach gekrümmten Schalenabschnitt oder einen sich in Richtung Hohlkörper radial nach außen konisch aufweitenden Schalenabschnitt aufweist, welcher in einer geschlossenen Stellung des Greiforgans (18) an einer distalen Seite des Greiforgans (18) angeordnet ist.

4. Behandlungsinstrument (4; 104; 204; 304; 404; 504; 604; 704; 804; 904; 1004) nach einem der Ansprüche 1 bis 3, **dadurch gekennzeichnet, dass** ein Abschnitt eines Randes eines proximalen Endes des Greiforgans (18) derart über ein Gelenk (24, 26; 824, 826; 924, 926) mit einem Abschnitt eines Randes eines distalen Endes des Hohlkörpers (16; 116; 216; 316; 416; 516; 616; 716; 1016) um eine Schwenkachse schwenkbeweglich verbunden ist, dass sich die Schwenkachse tangential zu beiden Abschnitten erstreckt.

5. Behandlungsinstrument (4; 104; 204; 304; 404; 504; 604; 804; 904; 1004; 1104) nach einem der Ansprüche 1 bis 4, **dadurch gekennzeichnet, dass** die Stellmechanik zumindest ein in dem Hohlkörper (16; 116; 216; 316; 416; 516; 616; 1016; 1116) verschiebbar gelagertes Stellglied (36; 136; 436, 437; 536, 537; 636, 637) aufweist, welches mit dem Greiforgan (18) über zumindest einen jeweils mit dem Stellglied (36; 136; 436, 437; 636, 637) und dem Greiforgan (18) schwenkbeweglich verbundenen Pleuel (32, 34) oder über zumindest ein jeweils mit dem Stellglied (36; 136) und dem Greiforgan (18) fest verbundenes flexibles Element (232, 234; 1132, 1134) verbunden ist und durch dessen Ansteuerung das Greiforgan (18) betätigbar ist.

6. Behandlungsinstrument (4; 104; 304; 404; 604; 804; 904; 1004) nach Anspruch 4 und 5, **dadurch gekennzeichnet, dass** der Pleuel (32, 34) derart über ein Gelenk (28, 30) mit einem weiteren Abschnitt des Randes des proximalen Endes des Greiforgans (18) um eine Schwenkachse schwenkbeweglich verbunden ist, dass sich die Schwenkachse senkrecht zu dem weiteren Abschnitt erstreckt.

7. Behandlungsinstrument (304; 604; 704) nach Anspruch 5 oder 6, **dadurch gekennzeichnet, dass**
das Greiforgan (18) zwei Greifteile (320, 322; 620, 622; 720, 722) aufweist,
eines der beiden Greifteile (322; 622; 722) fest mit dem Hohlkörper (316; 616; 716) verbunden ist,
das andere der beiden Greifteile (320; 620; 720) schwenkbeweglich oder schubbeweglich mit dem Hohlkörper (316; 616; 716) verbunden ist und
der zumindest eine Pleuel (332) oder das zumindest eine flexible Element das schwenkbeweglich oder schubbeweglich mit dem Hohlkörper (316; 616; 716) verbundene Greifteil (320; 620; 720) mit dem Stellglied (36; 636; 736) verbindet.

8. Behandlungsinstrument (604) nach Anspruch 7, **dadurch gekennzeichnet, dass**
das Behandlungsinstrument (604) ein weiteres Stellglied (637) aufweist,
ein weiterer Pleuel oder ein weiteres flexibles Element das schwenkbeweglich oder schubbeweglich mit dem Hohlkörper (616) verbundene Greifteil (620) mit dem weiteren Stellglied (637) verbindet und
ein distales Ende des Stellglieds (636) und ein distales Ende des weiteren Stellglieds (637) an zwei sich gegenüberliegenden Seiten des Hohlkörpers (616) angeordnet sind.

9. Behandlungsinstrument (4; 104; 204; 804; 904; 1004; 1104) nach Anspruch 5 oder 6, **dadurch gekennzeichnet, dass**
das Greiforgan (18) zwei Greifteile (20, 22; 1020, 1022; 1120, 1122) aufweist, welche schwenkbeweglich oder schubbeweglich mit dem Hohlkörper (16; 116; 216; 1016; 1116) verbunden sind,
der zumindest eine Pleuel (32) oder das zumindest eine flexible Element (232; 1132) das Stellglied (36; 136) mit einem der beiden Greifteile (20; 1020; 1120) verbindet und
ein weiterer Pleuel (34) oder ein weiteres flexibles Element (234; 1134) das Stellglied (36, 136) mit dem anderen der beiden Greifteile (22; 1022; 1122) verbindet.

10. Behandlungsinstrument (104) nach Anspruch 9, **dadurch gekennzeichnet, dass**
das Behandlungsinstrument (104) ein weiteres Stellglied (136) aufweist,
das weitere Stellglied (136) mit den beiden Greifteilen (20, 22) jeweils über einen weiteren Pleuel (32, 34) oder ein weiteres flexibles Element verbunden ist und
ein distales Ende des Stellglieds (36) und ein distales Ende des weiteren Stellglieds (136) an zwei sich gegenüberliegenden Seiten des Hohlkörpers (116) angeordnet sind.

11. Behandlungsinstrument (404) nach Anspruch 5 oder 6, **dadurch gekennzeichnet, dass**
das Greiforgan (18) zwei Greifteile (20, 22) aufweist, welche schwenkbeweglich oder schubbeweglich mit dem Hohlkörper (416) verbunden sind,
der zumindest eine Pleuel (32) oder das zumindest eine flexible Element das Stellglied (436) mit einem der beiden Greifteile (20) verbindet,
das Behandlungsinstrument (404) ein weiteres Stellglied (437) und einen weiteren Pleuel oder ein weiteres flexibles Element aufweist und
der weitere Pleuel oder das weitere flexible Element das weitere Stellglied (437) mit dem anderen der beiden Greifteile (22) verbindet.

12. Behandlungsinstrument (4; 104; 204; 404; 804; 904; 1004; 1104) nach einem der Ansprüche 9 bis 11, **dadurch gekennzeichnet, dass** die beiden Greifteile (20, 22; 1020, 1022; 1120, 1122) derart sich gegenüberliegend angeordnet sind, dass sich das Stellglied (36; 436) und/oder das weitere Stellglied (136; 437) symmetrisch zu/an/in einer mittig zwischen den beiden Greifteilen (20, 22; 1020, 1022; 1120, 1122) im geschlossenen Zustand liegenden Ebene erstrecken/erstreckt.

13. Behandlungsinstrument (1004) nach einem der Ansprüche 1 bis 12, **dadurch gekennzeichnet, dass** das Greiforgan (18) eine Aussparung (1056) mit einer maximalen Öffnungsweite von 1,1 mm oder weniger aufweist.

14. Endoskop (2) mit einem distalen Ende, welches mit einem Behandlungsinstrument (4; 104; 204; 304; 404; 504; 604; 704; 804; 904; 1004; 1104) nach einem der Ansprüche 1 bis 13 gekoppelt ist, wobei ein proximaler Abschnitt der Stellmechanik des Behandlungsinstruments (4; 104; 204; 304; 404; 504; 604; 704; 804; 904; 1004; 1104) entlang einer Außenseite des Endoskops (2) geführt ist.

15. Endoskop (2) mit einem distalen Ende, welches an einer Stirnfläche ein Objektiv (8) einer Optik aufweist und welches mit einem Behandlungsinstrument (4; 104; 204; 304; 404; 504; 604; 704; 804; 904; 1004; 1104) gekoppelt ist, wobei das Behandlungsinstrument (4; 104; 204; 304; 404; 504; 604; 704; 804; 904; 1004; 1104), einen Hohlkörper (16; 116; 216; 316; 416; 516; 616; 716; 1016; 1116) bildet, dessen Umfangswand innenseitig einen Hohlraum umgibt, sowie ein Greiforgan (18) aufweist, das Greiforgan (18) über eine Stellmechanik betätigbar ist, welche ein distales Ende aufweist, das sich innerhalb des Hohlkörpers (16; 116; 216; 316; 416; 516; 616; 716; 1016; 1116) befindet, welche sich von dem Hohlkörper (16; 116; 216; 316; 416; 516; 616; 716; 1016; 1116) aus in eine proximale Richtung erstreckt und welche einen proximalen Abschnitt aufweist, der entlang einer Außenseite des Endoskops (2) geführt werden kann,
wobei das Greiforgan (18) in Form einer Kappe ausgebildet ist, welche in einem an dem Endoskop (2) angebrachten Zustand des Behandlungsinstruments (4; 104; 204; 304; 404; 504; 604; 704; 804; 904; 1004; 1104) in eine geschlossene Stellung bringbar ist in welcher der Hohlraum an einem distalen Ende des Hohlkörpers zumindest mit Erreichen der geschlossenen Stellung vollständig verschließbar ist und in der das Objektiv (8) der Optik des Endoskops (2) in distaler Richtung von dem Greiforgan (18) verdeckt ist, und das Greiforgan (18) in einer geöffneten Stellung eine Stirnfläche des Hohlkörpers (16; 116; 216; 316; 416; 516; 616; 716; 1016; 1116) vollständig in distaler Richtung freigibt.

16. Endoskop (2) nach Anspruch 14 oder 15, **dadurch gekennzeichnet, dass** das Endoskop (2) einen Spülkanal aufweist, durch welchen Spülflüssigkeit zu einer an der Stirnfläche des Endoskops (2) angeordneten Spülkanalaustrittsöffnung förderbar ist, in dem an dem Endoskop (2) angebrachten Zustand des Behandlungsinstruments (4; 104; 204; 304; 404; 504; 604; 704; 804; 904; 1004; 1104) das Greiforgan (18) in der geschlossenen Stellung die Spülkanalaustrittsöffnung zumindest teilweise in distaler Richtung verdeckt und eine in proximale Richtung weisende Innenfläche des Greiforgans(18) in der geschlossenen Stellung des Greiforgans (18) derart von dem Objektiv (8) der Optik und von der Spülkanalaustrittsfläche beabstandet ist, dass bei einem Fördern von Spülflüssigkeit zu der Spülkanalaustrittsöffnung Spülflüssigkeit nach einem Austreten aus der Spülkanalaustrittsöffnung auf die in proximale Richtung weisende Innenfläche des Greiforgans (18) auftrifft und auf das Objektiv (8) der Optik reflektiert wird.

## Claims

1. A treatment instrument (4; 104; 204; 304; 404; 504; 604; 704; 804; 904; 1004; 1104) which is provided and configured to be coupled to a distal end or end portion of an endoscope (2) and which comprises the following elements:
- a substantially sleeve-shaped hollow body (16; 116; 216; 316; 416; 516; 616; 716; 1016; 1116) the circumferential wall of which internally surrounds an axially extending cavity and forms a proximal coupling portion, which is provided and configured to be attached to/plugged onto an endoscope shaft or endoscope head,
- a gripping element (18) that is supported on or articulated to a distal circumferential wall portion of the hollow body (16; 116; 216; 316; 416; 516; 616; 716; 1016; 1116) and
- an actuation mechanism provided for operating the gripping element (18) at least from a release position to a gripping position, said actuation mechanism having a proximal portion that extends from the hollow body (16; 116; 216; 316; 416; 516; 616; 716; 1016; 1116) to a proximal direction, and having a distal portion that is received in the cavity and comprises a distal end which is coupled to the gripping element (18) at the inner face thereof facing the cavity, wherein
- the gripping element (18) in the gripping position takes the shape of a cap; flap, cupola, door or roof by which the cavity is completely closed at a distal end of the hollow body (16; 116; 216; 316; 416; 516; 616; 716; 1016; 1116) at least when the gripping position is reached, and the gripping element (18), in the release position, completely releases an end face of the hollow body (16; 116; 216; 316; 416; 516; 616; 716; 1016; 1116) in the distal direction.

2. The treatment instrument (4; 104; 204; 304; 404; 504; 604; 704; 804; 904; 1004; 1104) according to claim 1, **characterized in that** the gripping element (18) is formed to be transparent at least in portions.

3. The treatment instrument (4; 104; 204; 304; 404; 504; 604; 704; 804; 904; 1004; 1104) according to claim 1 or 2, **characterized in that** the gripping element (18) comprises, at an end face, a flat plate portion, a single-curved shell portion or a shell portion conically widening radially outwards in the direction of the hollow body which is arranged, in a closed position of the gripping element (18), on a distal side of the gripping element (18).

4. The treatment instrument (4; 104; 204; 304; 404; 504; 604; 704; 804; 904; 1004) according to any one of the claims 1 to 3, **characterized in that** a portion of an edge of a proximal end of the gripping element (18) is connected to a portion of an edge of a distal end of the hollow body (16; 116; 216; 316; 416; 516; 616; 716; 1016) via a joint (24, 26; 824, 826; 924, 926) to be pivoting about a swivel axis such that the swivel axis extends tangentially to both portions.

5. The treatment instrument (4; 104; 204; 304; 404; 504; 604; 704; 804; 904; 1004; 1104) according to any one of the claims 1 to 4, **characterized in that** the actuation mechanism comprises at least one actuator (36; 136; 436, 437; 536, 537; 636, 637) movably supported in the hollow body (16; 116; 216; 316; 416; 516; 616; 1016; 1116) which is connected to the gripping element (18) via at least one connecting rod (32, 34) pivotally connected to respectively the actuator (36; 136; 436, 437; 636, 637) and the gripping element (18) or via at least one flexible element (232, 234; 1132, 1134) fixedly connected to respectively the actuator (36; 136) and the gripping element (18), and the gripping element (18) can be operated by driving the actuator.

6. The treatment instrument (4; 104; 304; 404; 604; 804; 904; 1004) according to claim 4 and 5, **characterized in that** the connecting rod (32, 34) is connected to be pivoting about a swivel axis to a further portion of the edge of the proximal end of the gripping element (18) via a joint (28, 30) such that the swivel axis is perpendicular to the further portion.

7. The treatment instrument (304; 604; 704) according to claim 5 or 6, **characterized in that**
the gripping element (18) comprises two gripping parts (320, 322; 620, 622; 720, 722),
one of the two gripping parts (322; 622; 722) is firmly connected to the hollow body (316; 616; 716),
the other one of the two gripping parts (320; 620; 720) is pivotally or slidingly connected to the hollow body (316; 616; 716) and
the at least one connecting rod (332) or the at least one flexible element connects the gripping part (320; 620; 720) that is pivotally or slidingly connected to the hollow body (316; 616; 716) to the actuator (36; 636; 736).

8. The treatment instrument (604) according to claim 7, **characterized in that**
the treatment instrument (604) comprises a further actuator (637),
a further connecting rod or a further flexible element connects the gripping part (620) that is pivotally or slidingly connected to the hollow body (616) to the further actuator (637), and
a distal end of the actuator (636) and a distal end of the further actuator (637) are arranged on two opposite sides of the hollow body (616).

9. The treatment instrument (4; 104; 204; 804; 904; 1004; 1104) according to claim 5 or 6, **characterized in that**
the gripping element (18) comprises two gripping parts (20, 22; 1020, 1022; 1120, 1122) which are pivotally or slidingly connected to the hollow body (16; 116; 216; 1016; 1116),
the at least one connecting rod (32) or the at least one flexible element (232; 1132) connects the actuator (36; 136) to one of the two gripping parts (20; 1020; 1120), and
a further connecting rod (34) or a further flexible element (234; 1134) connects the actuator (36, 136) to the other one of the two gripping parts (22; 1022; 1122).

10. The treatment instrument (104) according to claim 9, **characterized in that**
the treatment instrument (104) comprises a further actuator (136),
the further actuator (136) is connected to each of the two gripping parts (20, 22) via a further connecting rod (32, 34) or a further flexible element, and
a distal end of the actuator (36) and a distal end of the further actuator (136) are arranged on two opposite sides of the hollow body (116).

11. The treatment instrument (404) according to claim 5 or 6, **characterized in that**
the gripping element (18) comprises two gripping parts (20, 22) which are pivotally or slidingly connected to the hollow body (416),
the at least one connecting rod (32) or the at least one flexible element connects the actuator (436) to one of the two gripping parts (20),
the treatment instrument (404) comprises a further actuator (437) and a further connecting rod or a further flexible element, and
the further connecting rod or the further flexible element connects the further actuator (437) to the other of the two gripping parts (22).

12. The treatment instrument (4; 104; 204; 404; 804; 904; 1004; 1104) according to any one of the claims 9 to 11, **characterized in that** the two gripping parts (20; 22; 1020, 1022; 1120, 1122) are arranged to be opposed to each other so that the actuator (36; 436) and/or the further actuator (136; 437) extend(s) symmetrically to/on/in a plane situated centrally between the two gripping parts (20, 22; 1020, 1022; 1120, 1122) in the closed condition.

13. The treatment instrument (1004) according to any one of the claims 1 to 12, **characterized in that** the gripping element (18) comprises a recess (1056) with a maximum opening width of 1.1 mm or less.

14. An endoscope (2) having a distal end which is coupled to a treatment instrument (4; 104; 204; 304; 404; 504; 604; 704; 804; 904; 1004; 1104) according to any one of the claims 1 to 13, wherein a proximal portion of the actuation mechanism of the treatment instrument (4; 104; 204; 304; 404; 504; 604; 704; 804; 904; 1004; 1104) is guided along an outer face of the endoscope (2).

15. The endoscope (2) having a distal end which comprises a lens (8) of an optical system on an end face and which is coupled to a treatment instrument (4; 104; 204; 304; 404; 504; 604; 704; 804; 904; 1004; 1104), wherein the treatment instrument (4; 104; 204; 304; 404; 504; 604; 704; 804; 904; 1004; 1104) forms a hollow body (16; 116; 216; 316; 416; 516; 616; 716; 1016; 1116) the circumferential wall of which surrounds a cavity and comprises a gripping element (18), the gripping element (18) is operable via an actuation mechanism which comprises a distal end situated inside the hollow body (16; 116; 216; 316; 416; 516; 616; 716; 1016; 1116), which extends from the hollow body (16; 116; 216; 316; 416; 516; 616; 716; 1016; 1116) to a proximal direction and which comprises a proximal portion that can be guided along an outer face of the endoscope (2),
wherein
the gripping element (18) is configured in the form of a cap which, in a condition of the treatment instrument (4; 104; 204; 304; 404; 504; 604; 704; 804; 904; 1004; 1104) attached to the endoscope (2), can be brought into a closed position in which the cavity is completely closable at a distal end of the hollow body at least when the closed position is reached and in which the lens (8) of the optical system of the endoscope (2) is covered by the gripping element (18) in the distal direction, and the gripping element (18), in an opened position, completely releases an end face of the hollow body (16; 116; 216; 316; 416; 516; 616; 716; 1016; 1116) in the distal direction.

16. The endoscope (2) according to claim 14 or 15, **characterized in that** the endoscope (2) comprises a rinsing channel through which rinsing liquid can be conveyed to a rinsing channel outlet opening disposed on the end face of the endoscope (2), in the condition of the treatment instrument (4; 104; 204; 304; 404; 504; 604; 704; 804; 904; 1004; 1104) being attached to the endoscope (2), the gripping element (18) is covered at least partially in the distal direction in the closed position of the rinsing channel outlet opening, and an inner face of the gripping element (18) facing in the proximal direction is spaced apart from the lens (8) of the optical system and from the rinsing channel outlet surface in the closed position of the gripping element (18) such that, when rinsing liquid is conveyed to the rinsing channel outlet opening, rinsing liquid impinges on the inner face of the gripping element (18) facing the proximal direction after exiting the rinsing channel outlet opening and is reflected to the lens (8) of the optical system.

## Revendications

1. Instrument de traitement (4 ; 104 ; 204 ; 304 ; 404 ; 504 ; 604 ; 704 ; 804 ; 904 ; 1004 ; 1104) qui est prévu et réalisé pour être couplé à une extrémité distale ou une section terminale d'un endoscope (2) et qui présente les éléments suivants :
- un corps creux sensiblement en forme de douille (16 ; 116 ; 216 ; 316 ; 416 ; 516 ; 616 ; 716 ; 1016 ; 1116), dont la paroi périphérique entoure côté intérieur une cavité s'étendant axialement ainsi qu'une section de couplage proximale qui est prévue et réalisée pour être fixée/accrochée sur une tige d'endoscope ou une tête d'endoscope,
- un organe de saisie (18) qui est monté ou articulé au niveau d'une section de paroi périphérique du corps creux (16 ; 116 ; 216 ; 316 ; 416 ; 516 ; 616 ; 716 ; 1016 ; 1116) et
- un mécanisme de réglage prévue pour un actionnement de l'organe de saisie (18) au moins à partir d'une position de libération dans une position de saisie, mécanisme de réglage qui a une section proximale qui s'étend dans une direction proximale depuis le corps creux (16 ; 116 ; 216 ; 316 ; 416 ; 516 ; 616 ; 716 ; 1016 ; 1116) et qui a une section distale qui est logée dans la cavité et présente une extrémité distale qui est couplée à l'organe de saisie (18) au niveau de la face externe de celui-ci tournée vers la cavité, dans lequel
- l'organe de saisie (18) réalise dans la position de saisie la forme d'un cache, d'un clapet, d'un dôme, d'une porte ou d'un toit, avec lequel la cavité est fermée complètement au niveau d'une extrémité distale du corps creux (16 ; 116 ; 216 ; 316 ; 416 ; 516 ; 616 ; 716 ; 1016 ; 1116) au moins lorsque la position de saisie est atteinte et l'organe de saisie (18) libère complètement dans une position de libération une surface frontale du corps creux (16 ; 116 ; 216 ; 316 ; 416 ; 516 ; 616 ; 716 ; 1016 ; 1116) dans la direction distale.

2. Instrument de traitement (4 ; 104 ; 204 ; 304 ; 404 ; 504 ; 604 ; 704 ; 804 ; 904 ; 1004 ; 1104) selon la revendication 1, **caractérisé en ce que** l'organe de saisie (18) est réalisé au moins par sections de manière transparente.

3. Instrument de traitement (4 ; 104 ; 204 ; 304 ; 404 ; 504 ; 604 ; 704 ; 804 ; 904 ; 1004 ; 1104) selon la revendication 1 ou 2, **caractérisé en ce que** l'organe de saisie (18) présente au niveau d'une surface frontale une section de plaque plate, une section de coque simplement courbée ou une section de coque s'élargissant de manière conique radialement vers l'extérieur dans la direction du corps creux, qui est disposée dans une position fermée de l'organe de saisie (18) au niveau d'une face distale de l'organe de saisie (18).

4. Instrument de traitement (4 ; 104 ; 204 ; 304 ; 404 ; 504 ; 604 ; 704 ; 804 ; 904 ; 1004) selon l'une quelconque des revendications 1 à 3, **caractérisé en ce qu'**une section d'un bord d'une extrémité proximale de l'organe de saisie (18) est reliée avec mobilité pivotante à une section d'un bord d'une extrémité distale du corps creux (16 ; 116 ; 216 ; 316 ; 416 ; 516 ; 616 ; 716 ; 1016) par l'intermédiaire d'une articulation (24, 26 ; 824, 826 ; 924, 926) autour d'un axe de pivotement de telle sorte que l'axe de pivotement s'étend de manière tangentielle vers les deux sections.

5. Instrument de traitement (4 ; 104 ; 204 ; 304 ; 404 ; 504 ; 604 ; 804 ; 904 ; 1004 ; 1104) selon l'une quelconque des revendications 1 à 4, **caractérisé en ce que** le mécanisme de réglage présente au moins un actionneur (36 ; 136 ; 436, 437 ; 536, 537 ; 636, 637) logé de manière coulissante dans le corps creux (16 ; 116 ; 216 ; 316 ; 416 ; 516 ; 616 ; 1016 ; 1116), actionneur qui est relié à l'organe de saisie (18) par l'intermédiaire d'au moins une bielle (32, 34) reliée respectivement à l'actionneur (36 ; 136 ; 436, 437 ; 636, 637) et l'organe de saisie (18) ou par l'intermédiaire d'au moins un élément flexible (232, 234 ; 1132, 1134) relié de manière fixe respectivement à l'actionneur (36 ; 136) et à l'organe de saisie (18) et par la commande duquel l'organe de saisie (18) peut être actionné.

6. Instrument de traitement (4 ; 104 ; 304 ; 404 ; 604 ; 804 ; 904 ; 1004) selon les revendications 4 et 5, **caractérisé en ce que** la bielle (32, 34) est reliée par l'intermédiaire d'une articulation (28, 30) avec mobilité pivotante à une autre section du bord de l'extrémité proximale de l'organe de saisie (18) autour d'un axe de pivotement de telle sorte que l'axe de pivotement s'étend perpendiculairement à l'autre section.

7. Instrument de traitement (304 ; 604 ; 704) selon la revendication 5 ou 6, **caractérisé en ce que**
l'organe de saisie (18) présente deux parties de saisie (320, 322 ; 620, 622 ; 720, 722),
l'une des deux parties de saisie (322 ; 622 ; 722) est reliée de manière fixe au corps creux (316 ; 616 ; 716),
l'autre des deux parties de saisie (320 ; 620 ; 720) est reliée avec mobilité pivotante ou mobilité par poussée au corps creux (316 ; 616 ; 716) et
l'au moins une bielle (332) ou l'au moins un élément flexible relie la partie de saisie (320; 620; 720) reliée avec mobilité pivotante ou mobilité par poussée au corps creux (316 ; 616 ; 716) à l'actionneur (36 ; 636 ; 736).

8. Instrument de traitement (604) selon la revendication 7, **caractérisé en ce que**
l'instrument de traitement (604) présente un autre actionneur (637),
une autre bielle ou un autre élément flexible relie la partie de saisie (620) reliée avec mobilité pivotante ou mobilité par poussée au corps creux (616) à l'autre actionneur (637) et
une extrémité distale de l'actionneur (636) et une extrémité distale de l'autre actionneur (637) sont disposées au niveau de deux faces opposées du corps creux (616).

9. Instrument de traitement (4 ; 104; 204; 804; 904; 1004; 1104) selon la revendication 5 ou 6, **caractérisé en ce que**
l'organe de saisie (18) présente deux parties de saisie (20, 22 ; 1020, 1022 ; 1120, 1122) qui sont reliées avec mobilité pivotante ou mobilité par poussée au corps creux (16 ; 116 ; 216 ; 1016 ; 1116),
l'au moins une bielle (32) ou l'au moins un élément flexible (232 ; 1132) relie l'actionneur (36 ; 136) à l'une des deux parties de saisie (20 ; 1020 ; 1120) et
une autre bielle (34) ou un autre élément flexible (234 ; 1134) relie l'actionneur (36, 136) à l'autre des deux parties de saisie (22, 1022 ; 1122).

10. Instrument de traitement (104) selon la revendication 9, **caractérisé en ce que**
l'instrument de traitement (104) présente un autre actionneur (136),
l'autre actionneur (136) est relié aux deux parties de saisie (20, 22) respectivement par l'intermédiaire d'une autre bielle (32, 34) ou d'un autre élément flexible et
une extrémité distale de l'actionneur (36) et une extrémité distale de l'autre actionneur (136) sont disposées au niveau de deux faces opposées du corps creux (116).

11. Instrument de traitement (404) selon la revendication 5 ou 6, **caractérisé en ce que**
l'organe de saisie (18) présente deux parties de saisie (20, 22) qui sont reliées avec mobilité pivotante ou mobilité par poussée au corps creux (416),
l'au moins une bielle (32) ou l'au moins un élément flexible relie l'actionneur (436) à l'une des deux parties de saisie (20),
l'instrument de traitement (404) présente un autre actionneur (437) et une autre bielle ou un autre élément flexible et
l'autre bielle ou l'autre élément flexible relie l'autre actionneur (437) à l'autre des deux parties de saisie (22).

12. Instrument de traitement (4 ; 104 ; 204 ; 404 ; 804 ; 904 ; 1004 ; 1104) selon l'une quelconque des revendications 9 à 11, **caractérisé en ce que** les deux parties de saisie (20, 22 ; 1020, 1022 ; 1120, 1122) sont disposées de manière opposée l'une par rapport à l'autre de telle sorte que l'actionneur (36 ; 436) et/ou l'autre actionneur (136 ; 437) s'étendent de manière symétrique à/au niveau/dans un plan se trouvant à l'état fermé au centre entre les deux parties de saisie (20, 22 ; 1020, 1022 ; 1120, 1122).

13. Instrument de traitement (1004) selon l'une quelconque des revendications 1 à 12, **caractérisé en ce que** l'organe de saisie (18) présente un évidement (1056) avec une largeur d'ouverture maximale de 1,1 mm ou moins.

14. Endoscope (2) avec une extrémité distale qui est couplée à un instrument de traitement (4 ; 104 ; 204 ; 304 ; 404 ; 504 ; 604 ; 704 ; 804 ; 904 ; 1004 ; 1104) selon l'une quelconque des revendications 1 à 13, dans lequel une section proximale du mécanisme de réglage de l'instrument de traitement (4 ; 104 ; 204 ; 304 ; 404 ; 504 ; 604 ; 704 ; 804 ; 904 ; 1004 ; 1104) est guidée le long d'un côté extérieur de l'endoscope (2).

15. Endoscope (2) avec une extrémité distale qui présente un objectif (8) d'une optique au niveau d'une surface frontale et qui est couplée à un instrument de traitement (4 ; 104 ; 204 ; 304 ; 404 ; 504 ; 604 ; 704 ; 804 ; 904 ; 1004 ; 1104), dans lequel l'instrument de traitement (4 ; 104 ; 204 ; 304 ; 404 ; 504 ; 604 ; 704 ; 804 ; 904 ; 1004 ; 1104) forme un corps creux (16 ; 116 ; 216 ; 316 ; 416 ; 516 ; 616 ; 716 ; 1016 ; 1116) dont la paroi périphérique entoure côté intérieur une cavité, et présente un organe de saisie (18), l'organe de saisie (18) peut être activé par l'intermédiaire d'un mécanisme de réglage qui présente une extrémité distale qui se trouve à l'intérieur du corps creux (16 ; 116 ; 216 ; 316 ; 416 ; 516 ; 616 ; 716 ; 1016 ; 1116) qui s'étend dans une direction proximale à partir du corps creux (16 ; 116 ; 216 ; 316 ; 416 ; 516 ; 616 ; 716 ; 1016 ; 1116) et qui présente une section proximale qui peut être guidée le long d'un côté extérieur de l'endoscope (2),
dans lequel l'organe de saisie (18) est réalisé en forme de cache qui peut être mis dans une position fermée dans un état de l'instrument de traitement (4 ; 104 ; 204 ; 304 ; 404 ; 504 ; 604 ; 704 ; 804 ; 904 ; 1004 ; 1104) monté au niveau de l'endoscope (2), position dans laquelle la cavité peut être fermée complètement au niveau d'une extrémité distale du corps creux au moins lorsque la position fermée est atteinte et dans laquelle l'objectif (8) de l'optique de l'endoscope (2) est recouvert dans la direction distale par l'organe de saisie (18), et l'organe de saisie (18) libère complètement dans la direction distale dans une position ouverte une surface frontale du corps creux (16 ; 116 ; 216 ; 316 ; 416 ; 516 ; 616 ; 716 ; 1016 ; 1116).

16. Endoscope (2) selon la revendication 14 ou 15, **caractérisé en ce que** l'endoscope (2) présente un canal de rinçage à travers lequel un liquide de rinçage peut être transporté vers une ouverture de sortie de canal de rinçage disposé au niveau de la surface frontale de l'endoscope (2), dans lequel au niveau de l'état monté au niveau de l'endoscope (2) de l'instrument de traitement (4 ; 104 ; 204 ; 304 ; 404 ; 504 ; 604 ; 704 ; 804 ; 904 ; 1004 ; 1104) l'organe de saisie (18) recouvre au moins partiellement dans la direction distale l'ouverture de sortie de canal de rinçage et une surface intérieure de l'organe de saisie (18) orientée dans la direction proximale est écartée de l'objectif (8) de l'optique et de la surface de sortie de canal de rinçage dans la position fermée de l'organe de saisie (18) de telle sorte que lors d'un transport de liquide de rinçage vers l'ouverture de sortie de canal de rinçage un liquide de rinçage après une sortie hors de l'ouverture de sortie de canal de rinçage apparaît sur la surface intérieure de l'organe de saisie (18) orientée dans la direction proximale et est réfléchi sur l'objectif (8) de l'optique.
